# EUROPEAN PATENT APPLICATION

(11) **EP 4 706 811 A1**
(43) Date of publication of application: **11.03.2026**
(21) Application number: 24797253.2
(22) Date of filing: 27.02.2024
(51) Int. Cl.: B01J 19/08, C01B 3/34, H01J 37/32, B01D 53/32

(54) **GAS REFORMING SYSTEM**

(30) Priority: 27.04.2023 KR 20230055777
(71) Applicant: EN2CORE technology, Inc, Daejeon 34127 (KR)
(72) Inventor: KIM, Du-Eil, Seoul 01387 (KR); LEE, Yun-Seong, Sejong 30130 (KR); PARK, Jun-hyeok, Daejeon 34307 (KR); YANG, Seungjae, Daejeon 35234 (KR)
(74) Representative: Kim Kang, Jae Hee
(86) International application number: PCT/KR2024/002472
(87) International publication number: WO 2024/225591

(57) **Abstract**

A gas reforming system, which serves as an environmental energy solution, is proposed. More specifically, a gas reforming system utilizing plasma is proposed. A gas reforming system according to an embodiment may include a pre-treatment unit including a desulfurization process module and a first gas separation module, a plasma reforming unit including a discharge tube, an RF generation unit, an antenna structure, and an additional reaction module, and a post-treatment unit including a gas conversion module and a second gas separation module.

## Description

### Field of the Invention

The present disclosure relates generally to a gas reforming system and, more particularly, to a system for reforming a feed gas by means of plasma to obtain a target gas.

### Background Art

As interest in environmental issues has increased recently, research into environmental energy solutions that reform biogas or landfill gas into eco-friendly energy has been actively pursued.

In particular, various studies are being conducted on gas reforming methods utilizing catalysts, thermal energy, or plasma. Among these, inductively coupled plasma (ICP) technology offers several advantages over other methods. That is, it is more environmentally friendly, requires relatively shorter processing time, and allows for easier control of equipment.

However, the conventional ICP-based gas reforming method has not yet reached a level of energy conversion efficiency (ECE) sufficient to make it commercially feasible.

In this disclosure, it is aimed to introduce pre-treatment and post-treatment processes that reform a feed gas into a syngas using ICP, while interacting synergistically with plasma reforming. Through this approach, an efficient and environmentally friendly gas reforming system potentially suitable for practical and commercial applications is presented.

### Disclosure

### Technical Problem

Accordingly, the present disclosure has been made keeping in mind the above problems occurring in the related art, and an objective of the present disclosure is to produce a syngas having a specific composition ratio by reforming a feed gas by means of plasma.

An objective of the present disclosure is to design a pre-treatment process, a plasma reforming unit, and a post-treatment process in order to provide a gas reforming system with high energy conversion efficiency.

An objective of the present disclosure is to design a pre-treatment process such that the composition ratio of a reforming target gas supplied to a plasma reforming unit becomes a specific ratio.

An objective of the present disclosure is to provide a method for pre-activating a catalyst used in a gas conversion process during a post-treatment process.

An objective of the present disclosure is to design a structure that controls a gas flow path within a plasma reforming unit.

The objectives of the present disclosure are not limited to those mentioned above, and other objectives not mentioned will be clearly understood by those skilled in the art from the following description and the accompanying drawings.

### Technical Solution

In order to achieve the above objectives, according to one aspect of the present disclosure, there is provided a method for reforming gas comprises ① forming a first sub-feed stream and a second sub-feed stream by supplying a desulfurized feed gas to a first gas separation module, wherein the first separation module comprising a porous separation membrane, wherein the first sub-feed stream comprises the feed gases that do not pass through the porous separation membrane, wherein the second sub-feed stream comprises the feed gases that pass through the porous separation membrane, wherein a molar ratio of carbon dioxide in the first sub-feed stream is higher than a molar ratio of carbon dioxide in the second sub-feed stream, and wherein a molar ratio of methane in the first sub-feed stream is lower than a molar ratio of methane in the second sub-feed stream; ② forming a syngas comprising at least hydrogen and carbon monoxide by supplying the first sub-feed stream, the second sub-feed stream, and steam (H₂O) to a plasma reforming unit, wherein the first sub-feed stream is configured to be supplied to an upstream end of a plasma induction region defined by a discharge tube of the plasma reforming unit, wherein the second sub-feed stream and the steam is configured to be supplied to an downstream end of the plasma induction region, and wherein the first sub-feed stream, the second sub-feed stream and the steam is configured to be reformed by plasma that is generated in the plasma induction region, thereby forming the syngas; ③ forming an intermediate product by supplying the syngas to a gas conversion module, wherein, in an initial reduction section, the syngas is configured to be supplied to the gas conversion module through a first path in the initial reduction section, and the supplied syngas can be used as a reducing agent for the catalyst within the gas conversion module, wherein, in a main reaction section, the syngas is configured to be supplied to the gas conversion module through a second path and the supplied syngas can be converted to obtain the intermediate product, and wherein a pressure of the syngas supplied to the gas conversion module through the first path in the initial reduction section is lower than a pressure of the syngas supplied to the gas conversion module through the second path in the main reaction section; and ④ forming a final product by supplying the immediate product to a second gas separation module.

The technical solutions of the present disclosure are not limited to those mentioned above, and other technical solutions not mentioned will be clearly understood by those skilled in the art to which the present disclosure pertains from the following description and the accompanying drawings.

### Advantageous Effects

According to an embodiment, it is possible to provide a syngas having a required composition ratio depending on the type of final product to be produced through the system.

According to an embodiment, it is possible to provide a gas reforming system having an energy conversion efficiency sufficient to make it commercially feasible.

According to an embodiment, it is possible to achieve relatively low energy consumption during gas conversion in a post-treatment process.

According to an embodiment, it is possible to improve the gas reaction rate and the durability of components within a plasma reforming unit by controlling the gas flow within the plasma reforming unit.

The effects of the present disclosure are not limited to those mentioned above, and other effects not mentioned will be clearly understood by those skilled in the art from the following description and the accompanying drawings.

The above and other objectives, features, and other advantages of the present disclosure will be more clearly understood from the following detailed description when taken in conjunction with the accompanying drawings

### BRIEF DESCRIPTION OF THE DRAWINGS

FIG. 1 is a view illustrating a gas reforming system according to an embodiment;
FIG. 2 is a view illustrating a pre-treatment unit and a plasma reforming unit according to an embodiment;
FIG. 3 is a view illustrating a pre-treatment gas separation module according to an embodiment;
FIG. 4 is a view illustrating the configurations of a plasma reforming unit according to an embodiment;
FIG. 5 is a view illustrating an RF generation unit according to an embodiment;
FIG. 6 is a view illustrating an antenna structure according to an embodiment;
FIG. 7 is a view illustrating a main antenna module according to an embodiment;
FIG. 8 is a view illustrating a main antenna module according to another embodiment;
FIG. 9 is a view illustrating the internal design of a plasma reforming unit according to an embodiment;
FIG. 10 is a flowchart illustrating a plasma reforming method according to an embodiment;
FIG. 11 and FIG. 12 are views illustrating a process in which plasma reforming proceeds according to an embodiment;
FIG. 13 is a view illustrating a discharge tube and a guide structure according to an embodiment;
FIGS. 14 is a views illustrating a first gas supply nozzle and a second gas supply nozzle according to an embodiment;
FIG. 15A is a view illustrating an additional reaction module according to an embodiment;
FIG. 15B is a view illustrating insulating structures of the additional reaction module according to an embodiment;
FIG. 16 is a view illustrating a plasma reforming unit and a post-treatment unit according to an embodiment;
FIG. 17 is a view illustrating a structure for allowing a syngas to flow to a gas conversion module according to an embodiment;
FIG. 18 is a view illustrating a gas conversion method according to an embodiment; and
FIGS. 19 to 21 are views illustrating a process in which gas conversion proceeds according to an embodiment.

### DETAILED DESCRIPTION OF THE INVENTION

In some embodiments, a method for reforming gas may be provided. The method comprises ① forming a first sub-feed stream and a second sub-feed stream by supplying a desulfurized feed gas to a first gas separation module, wherein the first separation module comprising a porous separation membrane, wherein the first sub-feed stream comprises the feed gases that do not pass through the porous separation membrane, wherein the second sub-feed stream comprises the feed gases that pass through the porous separation membrane, wherein a molar ratio of carbon dioxide in the first sub-feed stream is higher than a molar ratio of carbon dioxide in the second sub-feed stream, and wherein a molar ratio of methane in the first sub-feed stream is lower than a molar ratio of methane in the second sub-feed stream; ② forming a syngas comprising at least hydrogen and carbon monoxide by supplying the first sub-feed stream, the second sub-feed stream, and steam (H₂O) to a plasma reforming unit, wherein the first sub-feed stream is configured to be supplied to an upstream end of a plasma induction region defined by a discharge tube of the plasma reforming unit, wherein the second sub-feed stream and the steam is configured to be supplied to an downstream end of the plasma induction region, and wherein the first sub-feed stream, the second sub-feed stream and the steam is configured to be reformed by plasma that is generated in the plasma induction region, thereby forming the syngas; ③ forming an intermediate product by supplying the syngas to a gas conversion module, wherein, in an initial reduction section, the syngas is configured to be supplied to the gas conversion module through a first path in the initial reduction section, and the supplied syngas can be used as a reducing agent for the catalyst within the gas conversion module, wherein, in a main reaction section, the syngas is configured to be supplied to the gas conversion module through a second path and the supplied syngas can be converted to obtain the intermediate product, and wherein a pressure of the syngas supplied to the gas conversion module through the first path in the initial reduction section is lower than a pressure of the syngas supplied to the gas conversion module through the second path in the main reaction section; and ④ forming a final product by supplying the immediate product to a second gas separation module.

In some embodiments, wherein forming the syngas comprises: igniting the plasma by supplying seed gas to the upstream end of the plasma induction region and by supplying power to an auxiliary antenna module configured to surround at least a portion of the discharge tube; and maintaining the plasma discharge by supplying power to a main antenna module configured to surround at least a portion of the discharge tube.

In some embodiments, wherein the seed gas is configured to be supplied to the plasma induction region from a first time to a second time, and wherein the first sub-feed stream is configured to be supplied to the plasma induction region from a third time after the first time to a fourth time after the second time. In some embodiments, wherein the second time at which the supply of seed gas is stopped is after the time at which power is supplied to the main antenna module. In some embodiments, wherein forming the syngas comprises controlling a flow rate of the first sub-feed stream, a flow rate of the second sub-feed stream and a flow rate of the steam such that a ratio of the methane, carbon dioxide and steam supplied to the plasma induction region is within a predetermined range; and wherein a molar ratio (H₂/CO) of hydrogen to carbon monoxide in the syngas is configured to be in the range 1.8 to 2.7. In some embodiments, wherein a direction in which the first sub-feed stream is supplied to the plasma induction region and a direction in which the second sub-feed stream is supplied to the plasma induction region are configured to be in opposite directions with respect to a central axis of the discharge tube. In some embodiments, performing an additional reaction on a syngas that is formed by plasma reforming by using an additional reaction module configured to be placed in a lower portion of the discharge tube, wherein the additional reaction module is configured to induce a catalytic reaction with the syngas such that a molar ratio of the hydrogen in the syngas can be increased. In some embodiments, wherein the gas conversion module is Water Gas Shift(WGS) module, and wherein the molar ratio of carbon monoxide in the intermediate product is lower than the molar ratio of carbon monoxide in the syngas. In some embodiments, wherein forming an intermediate product comprises cooling the syngas transferred to the first path by using a first heat exchanger module in the initial reduction section; and cooling and pressurizing the syngas transferred to the second path by using a second heat exchanger module and a compressor in the main reaction section. In some embodiments, wherein the first heat exchanger is configured to cool the supplied gas in a first temperature range, wherein the second heat exchanger is configured to cool the supplied gas in a second temperature range, and wherein a minimum value of the first temperature range is higher than a maximum value of the second temperature range. In some embodiments, wherein a first valve is configured to control the flow of the syngas to the first path, and wherein a second valve is configured to control the flow of the syngas to the second path. In some embodiments, wherein forming the syngas comprises,
inducing plasma in the plasma induction region by supplying power in the auxiliary antenna module and the main antenna module placed adjacent to the discharge tube, wherein an opening time of the first valve is configured to be within a predetermined time from the time at which power is supplied to the main antenna module. In some embodiments, wherein a closing time of the first valve is configured to be after a temperature in the gas conversion module satisfies a predetermined temperature. In some embodiments, wherein an opening time of the second valve is configured to be after a temperature in the gas conversion module satisfies a predetermined temperature. In some embodiments, wherein the closing time of the first valve is configured to be after the opening time of the second valve such that the initial reduction section and the main reaction section are configured to at least partially overlap. In some embodiments, wherein forming a final product comprises adsorbing a specific gas from the intermediate product by using an adsorbent in the second gas separation module.

Advantages and features of the disclosed embodiments and methods of accomplishing the same may be understood more readily by reference to the following detailed description of various embodiments and the accompanying drawings. Reference will now be made in detail to various embodiments of the present disclosure, specific examples of which are illustrated in the accompanying drawings and described below. The present disclosure may, however, be embodied in many different forms and should not be construed as being limited to only the embodiments set forth herein.

In the drawings, the thicknesses of layers and regions may be exaggerated for clarity. Further, when an element or layer is referred to as being "on" or "above" another element or layer, it can be directly on or above the other element or layer or intervening elements or layers may be present therebetween. In principle, like reference numerals designate like elements throughout the specification. In addition, like reference numerals are used to designate elements which have the same function within the same idea illustrated in the drawings of each embodiment, and overlapping descriptions thereof will be omitted.

Numerals (e.g., first, second, etc.) used in the description herein are merely identifiers for distinguishing one element from another element.

In addition, the suffixes "module" and "unit" which are used to refer to elements in the following embodiments are given or mixed together only considering the ease of creating the specification and have no meanings or roles that are distinguished from each other by themselves.

In the following embodiments, the singular forms "a", "an", and "the" are intended to include the plural forms as well, unless the context clearly indicates otherwise.

In the following embodiments, the terms "comprise", "include", "have", etc. specify the presence of stated features and/or elements but do not preclude the presence or addition of one or more other features, and or elements.

In the drawings, the sizes of elements may be exaggerated or reduced for convenience of explanation. For example, the sizes and thicknesses of elements in the drawings are arbitrarily illustrated for convenience of explanation, and embodiments of the present disclosure are not limited thereto.

When some example embodiments may be embodied otherwise, specific process steps described herein may be performed otherwise. That is, for example, two process steps described in a sequential order may be performed around the same time, or in reverse order.

In the following embodiments, when films, regions, elements, etc. are referred to as being connected to each other, they can be directly connected or be indirectly connected to each other with one or more intervening films, regions, elements, etc. interposed therebetween.

For example, when films, regions, elements, etc. are referred to as being electrically connected to each other, they can be electrically directly connected to each other, such as being physically combined or integrally formed to allow current flow, or be electrically indirectly connected to each other with one or more intervening films, regions, elements, etc. interposed therebetween.

In addition, for example, when films, regions, elements, etc. are referred to as being fluidly connected to each other, they can be fluidly directly connected to each other, such as being physically combined or integrally formed to allow fluid flow, or be fluidly indirectly connected to each other with one or more intervening films, regions, elements, etc. interposed therebetween.

### [Introduction]

The present disclosure relates generally to a gas reforming system and, more particularly, to a system for reforming a feed gas by means of plasma to obtain a target gas.

Plasma is a phase in which matter receives high energy and becomes separated into negatively charged electrons and positively charged ions, and it can be induced or generated through various methods.

Among many methods for generating plasma, an inductively coupled plasma (ICP) method creates plasma by supplying power to a coil or antenna, thereby forming an induced electric field or capacitive electric field in a specific space. This type of plasma is generally driven by a high-frequency power source, such as radio frequency (RF). Meanwhile, for convenience of explanation, plasma generated by a plasma generation system will be described hereinafter on the assumption that it is inductively coupled plasma (ICP). However, the technical idea of the present disclosure is not limited thereto.

Gas reforming refers to a process of converting an existing gas into a different type of gas, and it may be carried out using various methods, such as catalyst-based reforming, thermal energy-based reforming, and plasma-based reforming. Hereinafter, for convenience of explanation, gas reforming will be described as a process in which a supplied gas is converted into a different type of gas using plasma. However, the technical idea of the present disclosure is not limited thereto.

A feed gas refers to a gas that is to be reformed. For example, the feed gas may refer to a gas that is to be treated in a location requiring a gas reforming system. In another example, the feed gas may refer to a predetermined amount of gas collected using a specific method.

Depending on its origin, the feed gas may be classified as biogas, landfill gas (LFG), natural gas, or methane gas. Depending on its type, the feed gas may include methane (CH₄), carbon dioxide (CO₂), hydrogen (H₂), oxygen (O₂), nitrogen (N₂), and the like. Hereinafter, for convenience of explanation, the feed material gas will be described as including at least methane and carbon dioxide. However, the technical idea of the present disclosure is not limited thereto.

A target gas may refer to a final product (or final product gas) intended to be produced through a gas reforming system. The target gas may include, but is not limited to, high-purity hydrogen, methanol, aviation fuel, base oil, bionaphtha, bio-diesel, carbon black, graphene, carbon nanotubes, ammonia, or carbon monoxide.

### [Gas reforming system]

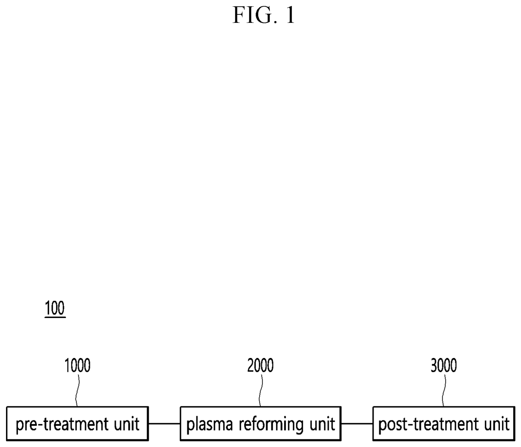

Hereinafter, a gas reforming system will be described with reference to FIG. 1.

FIG. 1 is a view illustrating a gas reforming system 100 according to an embodiment.

Referring to FIG. 1, the gas reforming system 100 may include a pre-treatment unit 1000, a plasma reforming unit 2000, and a post-treatment unit 3000.

The pre-treatment unit 1000 is a configuration for performing pre-treatment on a feed gas prior to gas reforming through the plasma reforming unit 2000. For example, the pre-treatment unit 1000 may perform a desulfurization process and a gas separation process for the feed gas. Specific details on the processes carried out in the pre-treatment unit 1000 will be described later.

The plasma reforming unit 2000 is a configuration for reforming gas using plasma. The plasma reforming unit 2000 may perform gas reforming by generating plasma through plasma induction and receiving the pre-treated feed gas, allowing it to pass through a plasma region.

For example, when reforming a feed gas containing methane and carbon dioxide, the following reactions may occur in the plasma reforming unit 2000.

CH₄ + H₂O →CO + 3H₂

CO + H₂O →CO₂ + H₂

CH₄ + CO₂ →2CO + 2H₂

The above reactions proceed as supplied gases are decomposed and recombined by the thermal energy of the plasma. As a result, methane and carbon dioxide in the feed gas are reformed by the plasma, producing a syngas containing hydrogen and carbon monoxide.

Here, the ratio of hydrogen to carbon monoxide in the reformed syngas may be determined depending on the molar ratio of gases supplied to the plasma reforming unit 2000.

At this time, the ratio of hydrogen to carbon monoxide in the syngas needs to be specified to a desirable value depending on the type of gas to be ultimately produced. For example, when the final product is high-purity hydrogen, a higher hydrogen ratio in the syngas is preferred. In another example, when the final product is methanol, the syngas preferably has a hydrogen-to-carbon monoxide ratio (H₂/CO) of about 2.6 and/or a H₂/(2CO+3CO₂) ratio of about 1.05. In yet another example, when the final product is aviation fuel, the syngas preferably has a hydrogen-to-carbon monoxide ratio (H₂/CO) of about 1.8 to about 2.2 and a relatively low amount of steam.

As described above, to adjust the ratio of hydrogen to carbon monoxide in the syngas, which varies depending on the type of final product, the molar ratio or flow rate of the gases supplied to the plasma reforming unit 2000 may be determined. This will be described in detail later.

The post-treatment unit 3000 is a configuration for treating the syngas reformed in the plasma reforming unit 2000 in order to obtain the final product. For example, the post-treatment unit 3000 may perform a gas conversion process to increase the purity of specific gases in the syngas and a gas separation process to separate a target gas.

Although not illustrated in FIG. 1, the gas reforming system 100 may further include a central control unit for controlling the pre-treatment unit 1000, the plasma reforming unit 2000, and the post-treatment unit 3000. The central control unit may control a first controller that controls the pre-treatment unit 1000, a second controller that controls the plasma reforming unit 2000, and a third controller that controls the post-treatment unit 3000.

In addition, although not illustrated in FIG. 1, the gas reforming system 100 may further include a collection unit for collecting a target gas or final product.

Hereinafter, each of the pre-treatment unit 1000, the plasma reforming unit 2000, and the post-treatment unit 3000 will be described in more detail with reference to the drawings.

### [Pre-treatment unit]

### 1. Structure of pre-treatment unit

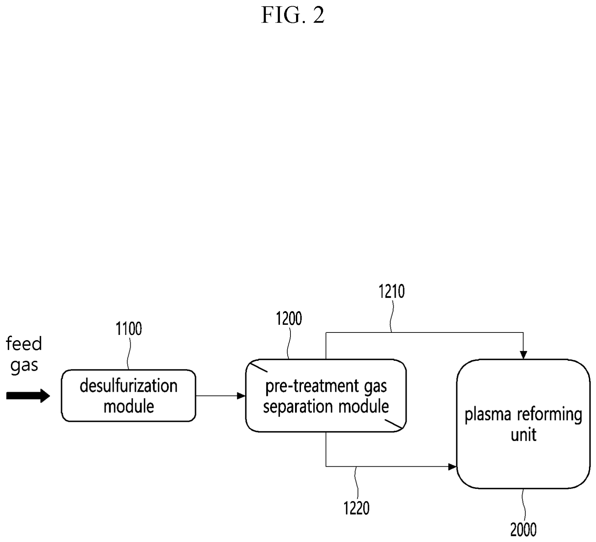

FIG. 2 is a view illustrating a pre-treatment unit 1000 and a plasma reforming unit 2000 according to an embodiment.

Referring to FIG. 2, the pre-treatment unit 1000 may include a desulfurization module 1100, a pre-treatment gas separation module 1200, a first gas separation pipe 1210, and a second gas separation pipe 1220.

The desulfurization module 1100 may perform a desulfurization process on a feed gas. The desulfurization process may be understood as one of the processes for removing unnecessary components from the feed gas before it is injected into the plasma reforming unit 2000. Specifically, when the feed gas is biogas, it may contain components such as hydrogen sulfide, halides, and silicon-containing compounds. These components need to be removed from the feed gas because they may form corrosive acids during the reforming process, which may deteriorate the durability of the plasma reforming unit 2000 or the post-treatment unit 3000.

The desulfurization module 1100 may receive the feed gas and remove hydrogen sulfide therefrom. The process of removing hydrogen sulfide in the desulfurization module 1100 may be classified into dry, wet, and biological processes. For example, the desulfurization module 1100 may perform a dry desulfurization process that removes hydrogen sulfide via physical/chemical adsorption using adsorbents such as metal oxides of iron, zinc, and copper, and activated carbon. In another example, The desulfurization module 1100 may perform a wet desulfurization process that brings the feed gas into contact with a liquid at a specific temperature and pressure to dissolve hydrogen sulfide. In yet another example, The desulfurization module 1100 may perform a biological desulfurization process that utilizes microbial metabolic activity to oxidize and decompose hydrogen sulfide for removal.

The desulfurization module 1100 may be fluidly connected to the pre-treatment gas separation module 1200. Specifically, an outlet of the desulfurization module 1100 may be fluidly connected to an inlet of the pre-treatment gas separation module 1200, allowing the gas that has undergone the desulfurization process in the desulfurization module 1100 to flow to the pre-treatment gas separation module 1200.

The pre-treatment unit 1000 may further include a module for removing specific components in addition to the desulfurization module 1100. For example, when the feed gas is landfill gas, the pre-treatment unit 1000 may further include a dehumidification module for removing moisture.

The pre-treatment gas separation module 1200 may separate a supplied gas into multiple sub-feed streams. For example, the pre-treatment gas separation module 1200 may separate a supplied feed stream into a first sub-feed stream and a second sub-feed stream. The process by which the pre-treatment gas separation module 1200 separates gases will be described later.

The pre-treatment gas separation module 1200 may be connected to multiple gas separation pipes. For example, an outlet of the pre-treatment gas separation module 1200 may be connected to the first gas separation pipe 1210 and the second gas separation pipe 1220. The first sub-feed stream separated in the pre-treatment gas separation module 1200 may flow to the first gas separation pipe 1210, while the second sub-feed stream may flow to the second gas separation pipe 1220.

The multiple gas separation pipes may be understood as a configuration that fluidly connects the pre-treatment gas separation module 1200 and the plasma reforming unit 2000 to each other. Here, each of the multiple gas separation pipes functions to supply a gas having a specific composition ratio to a specific location within the plasma reforming unit 2000. For example, as will be described below, the first gas separation pipe 1210 is configured to be connected to an inlet located at an upper end of the plasma reforming unit 2000 to supply carbon dioxide rich gas (CO₂ rich gas) to an upstream end of a plasma induction region of the plasma reforming unit 2000. In addition, as will also be described below, the second gas separation pipe 1220 is configured to be connected to a second inlet located at a lower end of the plasma reforming unit 2000 to supply methane rich gas (CH₄ rich gas) to a downstream end of the plasma induction region of the plasma reforming unit 2000.

### 2. Pre-treatment gas separation module

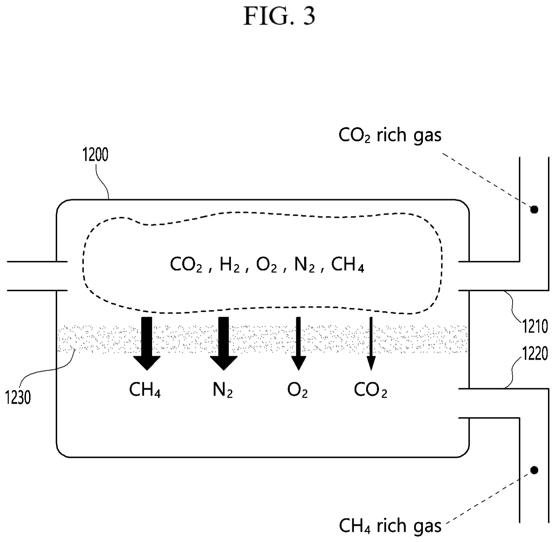

Hereinafter, a gas separation method performed in the pre-treatment gas separation module 1200 will be described with reference to FIG. 3.

FIG. 3 is a view illustrating a pre-treatment gas separation module 1200 according to an embodiment.

Referring to FIG. 3, a gas supplied to the pre-treatment gas separation module 1200 contains various types of gases. For example, the supplied gas may include carbon dioxide, hydrogen, oxygen, nitrogen, and methane.

Meanwhile, one of the critical aspects in plasma reforming is plasma induction and sustainment.

This is because stable plasma maintenance after induced can ensure consistent gas reforming or decomposition processes.

At this time, depending on the type of gas supplied to the plasma reforming unit 2000, plasma discharge may be either stably sustained or hindered. For example, in the case of a gas such as methane that participates in an endothermic reaction, it may be understood as a gas that hinders the sustainment of plasma discharge, because it absorbs thermal energy from the plasma. Therefore, in order to ensure the stability of plasma discharge, it is necessary to manage gases that hinder plasma discharge, such as methane gas, among the gases injected into the plasma reforming unit 2000.

In the gas reforming system 100 according to the present disclosure, when the gases are injected into the plasma reforming unit 2000, a gas that hinders plasma sustainment, such as methane gas, may be separated. The separated gas may then be injected into a downstream end of the plasma induction region so as not to pass through the plasma. At this time, such a plasma-inhibiting gas may also be decomposed by the heat from a plasma's tail, thereby ensuring stable plasma discharge while minimizing gas waste.

Referring again to FIG. 3, the pre-treatment gas separation module 1200 may include a separation membrane 1230. The separation membrane 1230 may have a porous structure. For example, the separation membrane 1230 may be understood as a membrane that includes pores having a predetermined size range.

The separation membrane 1230 may be disposed between an inlet of the pre-treatment gas separation module 1200 and the first gas separation pipe 1210. The separation membrane 1230 may be disposed between the inlet of the pre-treatment gas separation module 1200 and the second gas separation pipe 1220.

The separation membrane 1230 may separate gases having a predetermined particle size from among the gases in a feed stream supplied to the pre-treatment gas separation module 1200. For example, when the feed stream is supplied into the pre-treatment gas separation module 1200, a hydraulic pressure may be formed, and the resulting hydraulic pressure may force a portion of the feed stream to permeate through the separation membrane 1230.

Referring to FIG. 3, when the feed stream contains carbon dioxide and methane, methane having a relatively small particle size may permeate through the separation membrane 1230, while carbon dioxide having a relatively large particle size may not permeate through the separation membrane 1230.

As a result, the feed stream supplied to the pre-treatment gas separation module 1200 may be separated into a first sub-feed stream with a high molar ratio of carbon dioxide and a second sub-feed stream with a high molar ratio of methane. For example, the molar ratio of carbon dioxide to methane in the first sub-feed stream may be about 10:0 to 7:3, and the molar ratio of carbon dioxide to methane in the second sub-feed stream may be about 0:10 to 2:8.

Here, the molar ratio of carbon dioxide in the first sub-feed stream is greater than that of carbon dioxide in the second sub-feed stream. In addition, the molar ratio of methane in the first sub-feed stream is less than that of methane in the second sub-feed stream.

Meanwhile, the molar ratio of carbon dioxide in the first sub-feed stream and the molar ratio of methane in the second sub-feed stream may vary depending on the design method of the pre-treatment gas separation module 1200. For example, the molar ratios of carbon dioxide in the first sub-feed stream and methane in the second sub-feed stream may be determined based on the pressure in the pre-treatment gas separation module 1200 and the pore size of the separation membrane 1230.

As described above, i) in order to sustain plasma discharge, it is necessary to inject a gas involved in an endothermic reaction, such as methane, into the downstream end of the plasma induction region, and ii) as will be described below, in order to achieve a specific hydrogen-to-carbon monoxide ratio in a syngas reformed in the plasma reforming unit 2000, the composition ratio of gases introduced into the plasma reforming unit 2000 needs to be controlled to fall within a predetermined range. In view of this, the specifications of the separation membrane 1230 need to be appropriately selected to separate the feed stream supplied from the pre-treatment gas separation module 1200 into multiple sub-feed streams having desired gas composition ratios.

For example, when the feed stream supplied to the pre-treatment gas separation module 1200 contains about 34% carbon dioxide, 44% methane, 20% nitrogen, and 2% oxygen, the separation membrane 1230 may separate the feed stream such that the first sub-feed stream contains about 60% carbon dioxide, 25% methane, 12% nitrogen, and 3% oxygen, while the second sub-feed stream contains about 10% carbon dioxide, 58% methane, 29% nitrogen, and 3% oxygen.

In the above, the pre-treatment gas separation module 1200 has been described as being implemented in a form including the separation membrane 1230, but the technical idea of the present disclosure is not limited thereto.

For example, when a feed gas to be reformed in the gas reforming system 100 contains a large amount of nitrogen (which is considered an inhibitor of reforming efficiency in plasma reforming), nitrogen may be difficult to separate using the separation membrane 1230. In this case, the pre-treatment gas separation module 1200 may instead be implemented using a pressure swing adsorption (PSA) method.

Meanwhile, the pre-treatment unit 1000 may not further perform any special processes other than the aforementioned desulfurization and gas separation processes. For example, the pre-treatment unit 1000 may not perform a methane enrichment process that purifies methane in the feed gas. As a result, most of the feed gas may flow to the plasma reforming unit 2000 for reforming. Since no portion of the feed gas is discarded, energy conversion efficiency may be improved. Moreover, since no additional processes are required, the overall time required for gas reforming may be shortened, and operating costs associated with additional processes may be saved.

### [Plasma reforming unit]

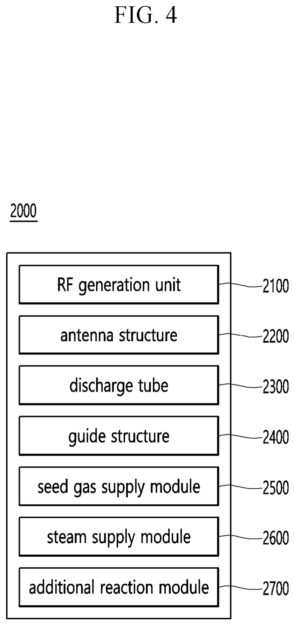

Hereinafter, the plasma reforming unit 2000 will be described with reference to FIGS. 4 to 15.

### 1. Configuration

FIG. 4 is a view illustrating the configurations of a plasma reforming unit 2000 according to an embodiment.

Referring to FIG. 4, the plasma reforming unit 2000 may include an RF generation unit 2100, an antenna structure 2200, a discharge tube 2300, a guide structure 2400, a seed gas supply module 2500, a steam supply module 2600, and an additional reaction module 2600.

First, the discharge tube 2300 defines a space where plasma is induced and a supplied gas is subjected to plasma reforming. In other words, the discharge tube 2300 includes an inner wall that defines a plasma induction region, and may have various forms, such as a cylindrical shape and a polygonal prism shape.

At least a portion of the discharge tube 2300 may be made of a heat-resistant material, such as at least one of aluminum oxide, silicon nitride, silicon nitride, silicon dioxide, yttrium oxide, ceramic, silicon carbide, and a combination thereof.

The antenna structure 2200 may be disposed around the discharge tube 2300. The antenna structure 2200 may function to generate an electromagnetic field within the discharge tube 2300 by receiving power. At this time, the component configured to supply power to the antenna structure 2200 is the RF generation unit 2100. The process by which the RF generation unit 2100 and the antenna structure 2200 induce plasma will be described later.

The RF generation unit 2100 may include multiple RF generators. For example, the RF generation unit 2100 may include the same number of RF generators as the number of antenna modules constituting the antenna structure 2200. Specifically, when the antenna structure 2200 includes an auxiliary antenna module and a main antenna module, the RF generation unit 2100 may include a first RF generator for applying power to the auxiliary antenna module and a second RF generator for applying power to the main antenna module. It goes without saying that even when the antenna structure 2200 includes multiple antenna modules, the RF generation unit 2100 may include a single RF generator, and power may be supplied to multiple antenna modules using this single RF generator.

The seed gas supply module 2500 may supply a seed gas into the discharge tube 2300. Here, the seed gas may be understood as a gas used to discharge plasma, such as argon gas or helium gas.

The steam supply module 2600 may supply steam into the discharge tube 2300. The steam supply module 2600 may be fluidly connected to the discharge tube 2300 to control the ratio of steam in a gas stream supplied into the discharge tube 2300 within a predetermined range.

The guide structure 2400 may supply a gas to a downstream end of a plasma induction region within the discharge tube 2300. For example, the guide structure 2400 may extend from a lower end of the discharge tube 2300 to the downstream end of the plasma induction region, allowing a gas supplied to the lower end of the discharge tube 2300 to flow along an outer wall of the guide structure 2400 and reach the downstream end of the plasma induction region. The guide structure 2400 may be coupled to the discharge tube 2300. To this end, the discharge tube 2300 may be divided into a first portion and a second portion having different widths. The shape of the guide structure 2400 and the positional relationship between the discharge tube 2300 and the guide structure 2400 will be described later.

The additional reaction module 2700 may induce an additional reaction for gases generated through plasma reforming. For example, the additional reaction module 2700 may include a catalyst and may induce a catalytic reaction of gases supplied after passing through the plasma induction region. As a result, the reforming efficiency in the plasma reforming unit 2000 may be improved by the additional reaction module 2700.

The additional reaction module 2700 may be disposed at the lower end of the discharge tube 2300. Alternatively, the additional reaction module 2700 may be disposed between the discharge tube 2300 and the post-treatment unit 3000 with respect to a gas flow path. The additional reaction module 2700 may be omitted. The configuration and design method of the additional reaction module 2700 will be described later.

### 2. RF generation unit

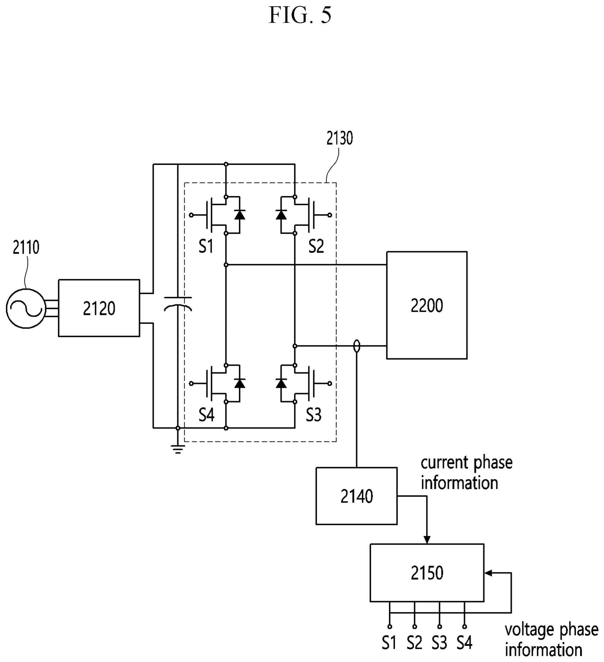

Hereinafter, the RF generation unit 2100 will be described in detail with reference to FIG. 5.

FIG. 5 is a view illustrating an RF generation unit 2100 according to an embodiment.

Referring to FIG. 5, the RF generation unit 2100 may include an AC power source 2110, a rectifier 2120, an inverter 2130, a sensor module 2140, and a control unit 2150. The RF generation unit 2100 may convert first alternating current (AC) power supplied from the AC power source 2110 into second alternating current (AC) power and supply it to a load. For example, the RF generation unit 2100 may convert the first AC power, which is conventionally used in households or industrial applications, into the second AC power, which has a frequency in the range of several hundred kHz to several tens of MHz and a magnitude of several kW or more, and provide it to the load.

Here, the load may include plasma generated by the antenna structure 2200 and the antenna structure 2200. In this case, the load may exhibit a resonant frequency that varies over time due to the induced plasma.

The rectifier 2120 may convert the output of the AC power source 2110 into direct current (DC). The rectifier 2120 may convert the first AC power supplied from the AC power source 2110 into DC power and apply it across both terminals of the inverter 2130. Meanwhile, in the present disclosure, the term "DC power" may be construed as meaning either a direct current or a direct voltage.

The inverter 2130 may receive the DC power from the rectifier 2120 and supply the second AC power to the load. For example, the inverter 2130 may receive a switch signal from the control unit 2150 and provide the second AC power to the load using the received switch signal.

The inverter 2130 may include at least one switch element controlled by a switch signal, and the second AC power supplied from the inverter 2130 to the load may have a driving frequency set based on the switch signal that the inverter 2130 receives from the control unit 2150.

For example, the inverter 2130 may be implemented in a full bridge form. Specifically, the inverter 1300 may include first to fourth switches S1, S2, S3, and S4. Here, the first to fourth switches S1, S2, S3, and S4 may be turned on or off in response to switch signals received from the control unit 1500. In this case, when the first and third switches S1 and S3 are turned on while the second and fourth switches S2 and S4 are turned off, a positive voltage may be applied to the load. On the contrary, when the first and third switches S1 and S3 are turned off while the second and fourth switches S2 and S4 are turned on, a negative voltage may be applied to the load. In this way, the inverter 2130 may alternately apply positive and negative voltages to the load, thereby applying AC power having a specific frequency.

The method of implementing the inverter 2130 is not limited to that described above. For example, the inverter 2130 may refer to a configuration including a circuit structure that performs a function of converting DC power into AC power.

The inverter 2130 may be controlled, for example, in a time delay type, a pulse width modulation (PWM) type, or a combination thereof, depending on frequency control methods of the control unit 2150.

Meanwhile, a capacitive element may be disposed between the rectifier 2120 and the inverter 2130. For example, the RF generation unit 2100 may include a capacitor connected in parallel to the rectifier 2120 and the inverter 2130, and the capacitor may discharge an AC component of power supplied to the inverter 2130 to a ground node GND.

The control unit 2150 may generate a switch signal. Specifically, the control unit 2150 may generate the aforementioned switch signal by receiving sensed data from the sensor module 2140, which will be described later. For example, the controller 2150 may be implemented to acquire data related to a resonant frequency, such as current and voltage of the load, from the sensor module 2140, and generate a switch signal based on the acquired data. Specifically, the control unit 2150 may acquire phase difference data or a delay time using phase data of the current applied to the load and phase data of the voltage applied to the load, which are acquired from the sensor module 2140, and generate a switch signal based on the phase delay data or the delay time.

The control unit 2150 may be implemented as a central processing unit (CPU), microprocessor, processor core, multiprocessor, application-specific integrated circuit (ASIC), field-programmable gate array (FPGA), or the like, depending on hardware, software, or a combination thereof.

The sensor module 2140 may provide data related to the resonant frequency of the load or data related to the power supplied to the load to the control unit 2150.

Although not illustrated in FIG. 5, the sensor module 2140 may include a transformer, a filter, and a comparator.

The sensor module 2140 may receive a current or voltage signal flowing to the load and convert it into a current or voltage signal of a different magnitude using the current transformer, filter the converted current or voltage signal through the filter, and output phase data to the control unit 2150 through the comparator.

The current transformer may be inductively coupled to a wire between the inverter 2130 and the load, and may convert the voltage or current signal applied to the load and provide the converted voltage or current signal to the filter. Specifically, the current transformer may convert current flowing through a conductive wire connected to the load into a voltage signal.

The filter may remove a DC component from the input current or voltage signal and output it to the comparator. To this end, the filter may perform high-pass band filtering or low-pass band filtering.

The comparator may acquire phase data. For example, the comparator may acquire phase data by comparing a voltage signal acquired from the current transformer or the filter with a predetermined value. The phase data may refer to phase data of current applied to the load.

At least one of the configurations included in the sensor module 2140 may be omitted, and may also be implemented in other ways.

As described above, the RF generation unit 2100 may control the driving frequency of the second AC power applied to the load based on data related to the resonant frequency of the load. In other words, the RF generation unit 2100 may output the driving frequency of the second AC power to match the resonant frequency of the load by tracking the load's resonant frequency that shifts with changes in plasma conditions. Accordingly, unnecessary power consumption may be prevented and the durability of the plasma system may be improved.

Meanwhile, although not illustrated in FIG. 5, the RF generation unit 2100 may include a memory. The memory may store various data. Various data may be temporarily or semipermanently stored in the memory. Examples of the memory may include a hard disk drive (HDD), a solid state drive (SSD), a flash memory, a read-only memory (ROM), and a random access memory. The memory may be implemented in a built-in form within the RF generation unit 2100 or in a detachable form.

In addition, the RF generation unit 2100 may include an input unit for receiving an input from a user. The input unit may receive a user input from the user. The user input may be implemented in various forms, including a key input, a touch input, and a voice input. The input unit is a comprehensive concept that encompasses not only a traditional type of keypad, keyboard, and mouse, but also a touch sensor that detects user's touch, and various types of input devices that detect or receive various types of user inputs.

In addition, the RF generation unit 2100 may include an output unit for providing information to the user. The output unit may output information about the status of the plasma reforming unit 2000 (e.g., sensor values measured by the sensor module 2140, driving frequency of the RF generation unit 2100, temperature of the antenna structure 2200, and the like) and provide this information to the user. The output unit is a comprehensive concept that encompasses a display that outputs a video, a speaker that outputs sound, a haptic device that generates vibration, and various other types of output devices.

The RF generation unit 2100 described above may have at least one of its configurations omitted. For example, the RF generation unit 2100 may not include the sensor module 2140, and instead acquire electrical data related to the load from an external sensor. In another example, the RF generator unit 2100 may not include the AC power source 2110 and the rectifier 2120, and instead receive DC power or rectified DC power from an external source.

### 3. Antenna structure

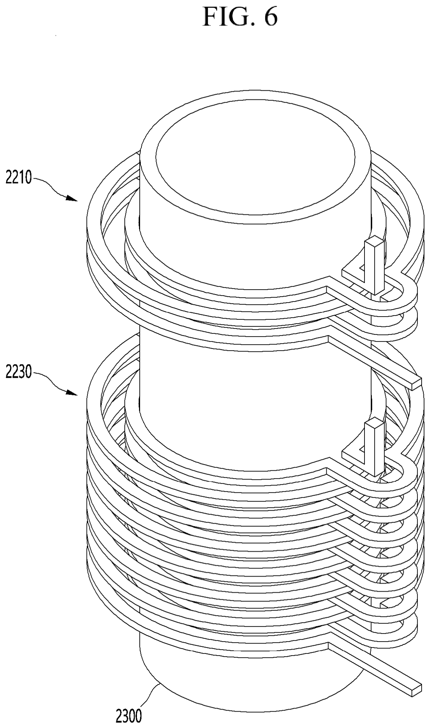

Hereinafter, the antenna structure 2200 will be described in detail with reference to FIGS. 6 to 8.

FIG. 6 is a view illustrating an antenna structure 2200 according to an embodiment.

Referring to FIG. 6, the antenna structure 2200 may include an auxiliary antenna module 2210 and a main antenna module 2230. The auxiliary antenna module 2210 may be understood as an antenna module for igniting plasma, and the main antenna module 2230 may be understood as an antenna module for sustaining the ignited plasma. The process by which plasma is ignited and sustained will be described later.

The auxiliary antenna module 2210 may be disposed around the discharge tube 2300. The auxiliary antenna module 2210 may be implemented in a coil-like shape or ring shape that surrounds an outer surface of the discharge tube 2300.

The auxiliary antenna module 2210 may have a layered structure. The auxiliary antenna module 2210 may have a structure in which identical or similar structures are stacked in a longitudinal direction of the discharge tube 2300. For example, as illustrated in FIG. 6, the auxiliary antenna module 2210 may have a two-layer structure including two layer antennas. The number of layers of the auxiliary antenna module 2210 is not limited to two and may be appropriately determined as needed.

Each layer of the auxiliary antenna module 2210 may be composed of multiple turns. For example, as illustrated in FIG. 6, the auxiliary antenna module 2210 may be composed of two turn antennas: an inner turn antenna that surrounds the outer surface of the discharge tube 2300 and an outer turn antenna that surrounds the inner turn antenna. The number of turns constituting each layer the auxiliary antenna module 2210 is not limited to two and may be appropriately determined as needed.

The main antenna module 2230 may be disposed around the discharge tube 2300. The main antenna module 2230 may be implemented in a coil-like shape or ring shape that surrounds the outer surface of the discharge tube 2300.

The main antenna module 2230 may have a layered structure. The main antenna module 2230 may have a structure in which identical or similar structures are stacked in the longitudinal direction of the discharge tube 2300. For example, as illustrated in FIG. 6, the main antenna module 2230 may have a seven-layer structure. The number of layers of the main antenna module 2230 is not limited to seven and may be appropriately determined as needed.

Each layer of the main antenna module 2230 may be composed of multiple turns. For example, as illustrated in FIG. 3, the main antenna module 2230 may be composed of two turn antennas: an inner turn antenna that surrounds the outer surface of the discharge tube 2300 and an outer turn antenna that surrounds the inner turn antenna. The number of turns constituting each layer of the main antenna module 2230 is not limited to two and may be appropriately determined as needed.

The main antenna module 2230 may have at least one capacitive element. For example, a capacitive element may be electrically interposed between multiple antennas constituting the main antenna module 2230. Specifically, when the main antenna module 2230 includes multiple layer antennas and each layer antenna includes multiple turn antennas, a capacitive element may be electrically interposed between the multiple layer antennas and/or between the multiple turn antennas.

Here, the capacitive element may refer to an element or its equivalent circuit, the element having a function of storing electrical energy, such as a capacitor, a ceramic capacitor, a multilayer ceramic capacitor, or an ultracapacitor.

Unlike the main antenna module 2230, the auxiliary antenna module 2210 may not include a capacitive element. This is because the inclusion of the capacitive element in the antenna module tends to lower voltage applied across both terminals of the antenna module, whereas, as will be described below, a relatively high voltage needs to be applied to the auxiliary antenna module 2210 during plasma induction. It goes without saying that the auxiliary antenna module 2210 may include a capacitive element, and the main antenna module 2230 may not include a capacitive element.

The auxiliary antenna module 2210 and the main antenna module 2230 may be disposed around the discharge tube 2300 at a predetermined distance apart from each other. For example, as illustrated in FIG. 6, the main antenna module 2230 may be spaced apart from the auxiliary antenna module 2210 by a predetermined distance along the longitudinal direction of the discharge tube 2300.

Meanwhile, the energy conversion efficiency (ECE) in the plasma reforming unit 2000 may vary depending on the shape of the main antenna module 2230. Here, the ECE may refer to the degree to which gases in a feed stream supplied to the plasma reforming unit 2000 are converted into a syngas through plasma reforming. Specifically, the ECE may be obtained as a ratio of the energy of gases converted in the plasma reforming unit 2000 to the energy of gases injected into the plasma reforming unit 2000. Meanwhile, in calculating the ECE, the amount of electrical energy used in the plasma reforming unit 2000 may also be taken into consideration.

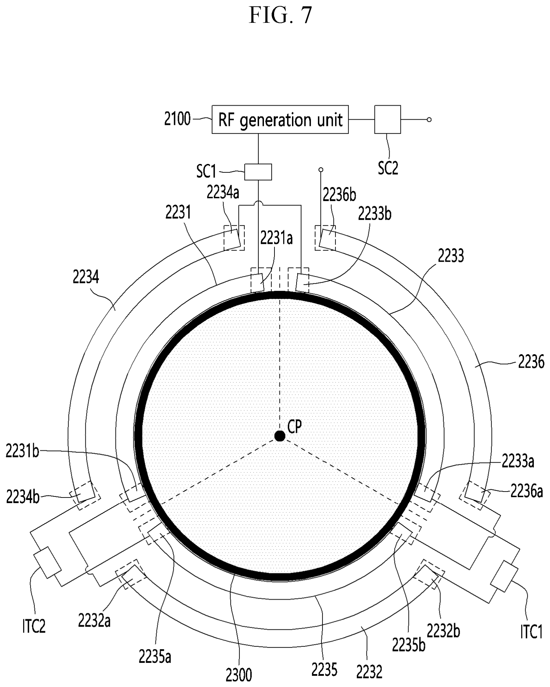

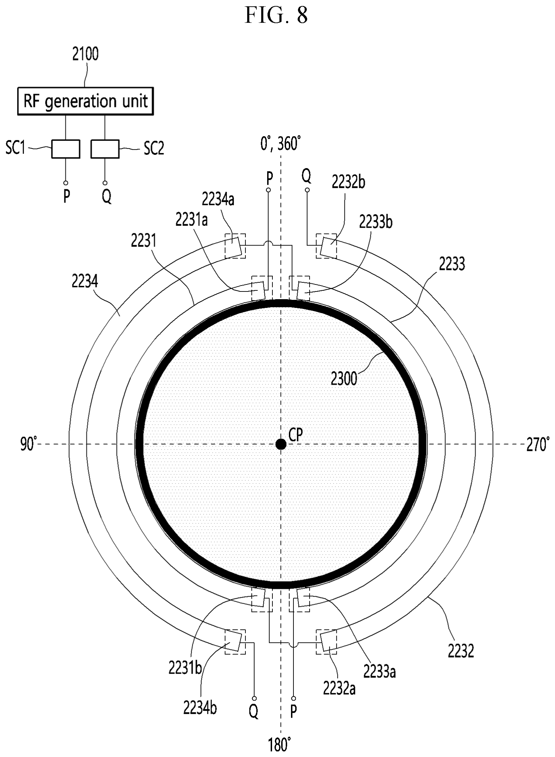

Hereinafter, the shape of the main antenna module 2230 for increasing the ECE will be described with reference to FIGS. 7 and 8.

FIG. 7 is a view illustrating a main antenna module 2230 according to a first embodiment.

Referring to FIG. 7, the main antenna module 2230 may be composed of multiple antenna segments. For example, the main antenna module 2230 may include first to third inner antenna segments 2231, 2233, and 2235 forming an inner turn, and first to third outer antenna segments 2232, 2234, and 2236 forming an outer turn.

The first inner antenna segment 2231 may be electrically connected to the first outer antenna segment 2232. The other end 2231b of the first inner antenna segment may electrically connect to one end 2232a of the first outer antenna segment. The other end 2231b of the first inner antenna segment may be electrically connected to the one end 2232a of the first outer antenna segment via a first connector. Here, the first connector may be implemented as a U-shaped wire or coil, but is not limited thereto.

Meanwhile, one end 2231a of the first inner antenna may electrically connect to one end of the RF generation unit 2100. At this time, a first auxiliary capacitive element SC1 may be electrically interposed between the one end 2231a of the first inner antenna segment and the RF generation unit 2100.

The first outer antenna segment 2232 may be electrically connected to the second inner antenna segment 2233. The other end 2232b of the first outer antenna segment may electrically connect to one end 2233a of the second inner antenna segment. The other end 2232b of the first outer antenna segment may be connected to the one end 2233a of the second inner antenna segment via a first inter-turn capacitive element ITC1.

The second inner antenna segment 2233 may be electrically connected to the second outer antenna segment 2234. The other end 2233b of the second inner antenna segment may electrically connect to one end 2234a of the second outer antenna segment. The other end 2233b of the second inner antenna segment may be electrically connected to the one end 2234a of the second outer antenna segment via a second connector. Here, the second connector may be implemented as a U-shaped wire or coil, but is not limited thereto.

The second outer antenna segment 2234 may be electrically connected to the third inner antenna segment 2235. The other end 2234b of the second outer antenna segment may electrically connect to one end 2235a of the third inner antenna segment. The other end 2234b of the second outer antenna segment may be connected to the one end 2235a of the third inner antenna segment 2235 via a second inter-turn capacitive element ITC2.

The third inner antenna segment 2235 may be electrically connected to the third outer antenna segment 2236. The other end 2235b of the third inner antenna segment may electrically connect to one end 2236a of the third outer antenna segment. The other end 2235b of the third inner antenna segment 2235 may be electrically connected to the one end 2236a of the third outer antenna segment 2236 via a third connector. Here, the third connector may be implemented as a U-shaped wire or coil, but is not limited thereto.

Meanwhile, the other end 2236b of the third outer antenna segment may be electrically connected to an antenna segment of another layer. However, when the main antenna module 2230 is composed of a single layer, the other end 2236b of the third outer antenna segment may be electrically connected to the other end of the RF generation unit 2100. At this time, a second auxiliary capacitive element SC2 may be electrically interposed between the other end 2236b of the third outer antenna segment 2236 and the RF generation unit 2100. However, either one of the first auxiliary capacitive element SC1 or the second auxiliary capacitive element SC2 may be omitted.

When the antenna segments within the main antenna module 2230 are connected as described above, they may be connected in series in the following order with respect to the RF generation unit 2100: the first inner antenna segment 2231, the first outer antenna segment 2232, the first inter-turn capacitive element ITC1, the second inner antenna segment 2233, the second outer antenna segment 2234, the second inter-turn capacitive element ITC2, the third inner antenna segment 2235, and the third outer antenna segment 2236.

Here, the first outer antenna segment 2232 and the first inter-turn capacitive element ITC1 may be electrically interposed between the first inner antenna segment 2231 and the second inner antenna segment 2233.

In addition, the second outer antenna segment 2234 and the second inter-turn capacitive element ITC2 may be electrically interposed between the second inner antenna segment 2233 and the third inner antenna segment 2235.

In addition, the second inter-turn capacitive element ITC2 and the third inner antenna segment 2235 may be electrically interposed between the second outer antenna segment 2234 and the third outer antenna segment 2236.

The portion illustrated in FIG. 7 represents a single layer antenna. The main antenna module 2230 may be composed of multiple layer antennas, each of which may be implemented as illustrated in FIG. 7. In this case, a third outer antenna segment of a first layer antenna may be electrically connected to a first inner antenna segment of a second layer antenna, which is the next layer following the first layer antenna, and an inter-layer capacitor may be electrically interposed therebetween.

FIG. 8 is a view illustrating a main antenna module 2230 according to a second embodiment.

Referring to FIG. 8, the main antenna module 2230 may be composed of multiple antenna segments. For example, the main antenna module 2230 may include a first inner antenna segment 2231 and a second inner antenna segment 2233 forming an inner turn, and a first outer antenna segment 2232 and a second outer antenna segment 2234 forming an outer turn.

The first inner antenna segment 2231 may be electrically connected to the first outer antenna segment 2232, and the second inner antenna segment 2233 may be electrically connected to the second outer antenna segment 2234. The manner in which the first inner antenna segment 2231 is connected to the first outer antenna segment 2232 and the manner in which the second inner antenna segment 2233 is connected to the second outer antenna segment 2234 are the same as those described in FIG. 7 and will thus be omitted.

Meanwhile, the other end 2232b of the first outer antenna segment 2232 may be connected to the first inner antenna segment of another layer antenna, or to the RF generation unit 2100 when the main antenna module 2230 is composed of a single layer. Also, the other end 2234b of the second outer antenna segment 2234 may be connected to the second inner antenna segment of another layer antenna, or to the RF generation unit 2100 when the main antenna module 2230 is composed of a single layer. In this case, an inter-layer capacitor may be electrically interposed between the antenna segments of different layer antennas.

In other words, in the main antenna module 2230 according to the second embodiment, the first inner antenna segments and the first outer antenna segments of each layer may be connected in series with each other to form one closed circuit with the RF generation unit 2100, and the second inner antenna segments and the second outer antenna segments of each layer may be connected in series with each other to form another closed circuit with the RF generation unit 2100.

### 4. Plasma reforming method

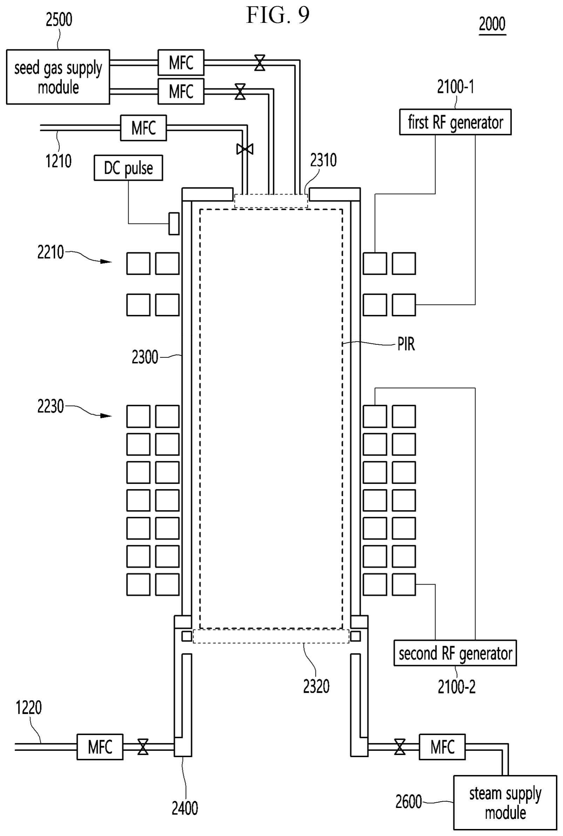

Hereinafter, a plasma reforming method will be described with reference to FIGS. 9 to 12.

Prior to describing the plasma reforming method, the internal design of the plasma reforming unit 2000 for reforming will first be described.

FIG. 9 is a view illustrating the internal design of a plasma reforming unit 2000 according to an embodiment.

First, an antenna structure 2200 may be disposed to surround a discharge tube 2300. At this time, a region within the discharge tube 2300 that corresponds to a portion surrounded by the antenna structure 2200 may be understood as a plasma induction region PIR.

As described above, the antenna structure 2200 may be electrically connected to an RF generation unit 2100. The RF generation unit 2100 may include a first RF generator 2100-1 corresponding to an auxiliary antenna module 2210 of the antenna structure 2200, and a second RF generator 2100-2 corresponding to a main antenna module 2230 of the antenna structure 2200.

Specifically, the auxiliary antenna module 2210 may be electrically connected to the first RF generator 2100-1. One end of the auxiliary antenna module 2210 may be electrically connected to one end of the first RF generator 2100-1, while the other end of the auxiliary antenna module 2210 may be electrically connected to the other end of the first RF generator 2100-1.

In addition, the main antenna module 2230 may be electrically connected to the second RF generator 2100-2. One end of the main antenna module 2230 may be electrically connected to one end of the second RF generator 2100-2, while the other end of the main antenna module 2230 may be electrically connected to the other end of the second RF generator 2100-2. At this time, a capacitive element may be electrically interposed between the one end of the main antenna module 2230 and the one end of the second RF generator 2100-2 and/or between the other end of the main antenna module 2230 and the other end of the second RF generator 2100-2.

In addition, a DC pulse power supply and a DC electrode may be disposed on the outer side of the discharge tube 2300. The DC electrode may receive a pulse voltage from the DC pulse power supply to generate a strong electric field inside the discharge tube 2300. The DC pulse power supply and DC electrode may be utilized for plasma ignition, as will be described below.

The discharge tube 2300 may have an inlet for supplying a feed stream. Multiple inlets may be formed in the discharge tube 2300.

For example, the discharge tube 2300 may include a first inlet 2310 and a second inlet 2320. The plasma induction region PIR may be disposed between the first inlet 2310 and the second inlet 2320. Specifically, the first inlet 2310 may be positioned at an upstream end of the plasma induction region PIR, and the second inlet 2320 may be positioned at a downstream end of the plasma induction region PIR.

At this time, the first inlet 2310 may be fluidly connected to the first gas separation pipe 1210 of the pre-treatment unit 1000 and a seed gas supply module 2500. In other words, a first sub-feed stream flowing through the first gas separation pipe 1210 and a seed gas supplied from the seed gas supply module 2500 may flow into the plasma induction region PIR through the first inlet 2310. Although FIG. 9 illustrates that the seed gas and the first sub-feed stream flow to the first inlet 2310 through separate pipes, the technical idea of the present disclosure is not limited thereto. For example, the seed gas and the first sub-feed stream may also flow to the first inlet 2310 through the same pipe.

A swirl generator may be disposed at the first inlet 2310 such that a gas supplied into the discharge tube 2300 forms a swirl. The swirl generator may rotate the supplied gas in a clockwise or counterclockwise direction before supplying it into the discharge tube 2300.

In addition, the second inlet 2310 may be fluidly connected to the second gas separation pipe 1220 of the pre-treatment unit 1000 and a steam supply module 2600. In other words, a second sub-feed stream flowing through the second gas separation pipe 1220 and steam supplied from the steam supply module 2600 may flow into the plasma induction region PIR through the second inlet 2320. Although FIG. 9 illustrates that the second sub-feed stream and the steam are supplied through separate pipes, the technical idea of the present disclosure is not limited thereto. For example, the second sub-feed stream and the steam may also flow to the second inlet 2320 through the same pipe.

Meanwhile, the flow rates of the first sub-feed stream and the seed gas introduced into the first inlet 2310 may be controlled. For example, flow rate sensors may be installed at arbitrary points along pipes through which the first sub-feed stream and the seed gas flow, and the flow rates of the first sub-feed stream and the seed gas may be controlled based on the detected flow rates. A mass flow controller (MFC) may be utilized for flow rate control.

Similarly, the flow rates of the second sub-feed stream and the steam introduced into the second inlet 2320 may also be controlled.

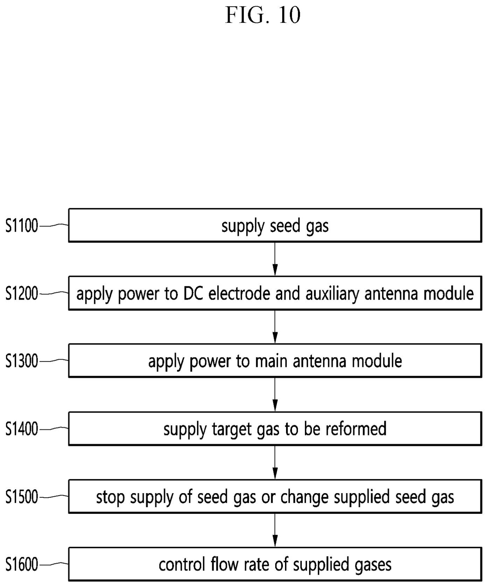

Hereinafter, a plasma reforming method will be described with reference to FIGS. 10 to 12.

FIG. 10 is a flowchart illustrating a plasma reforming method according to an embodiment.

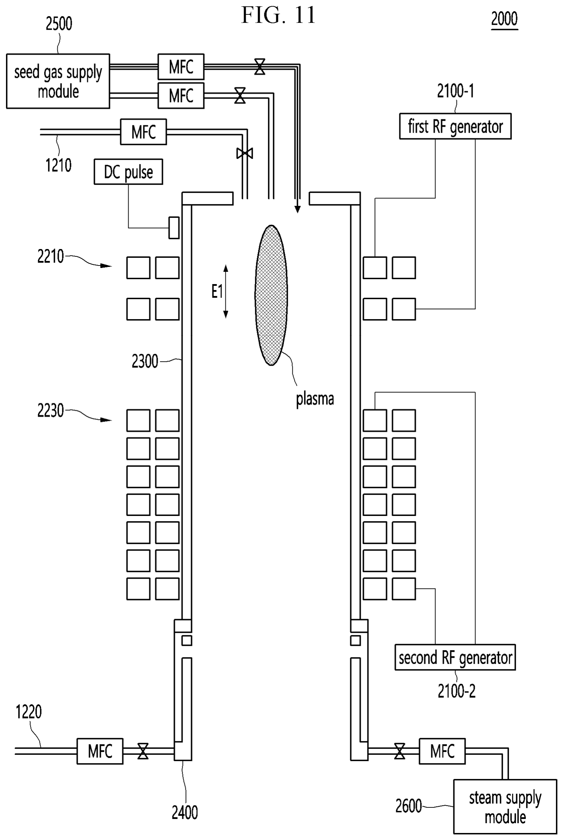

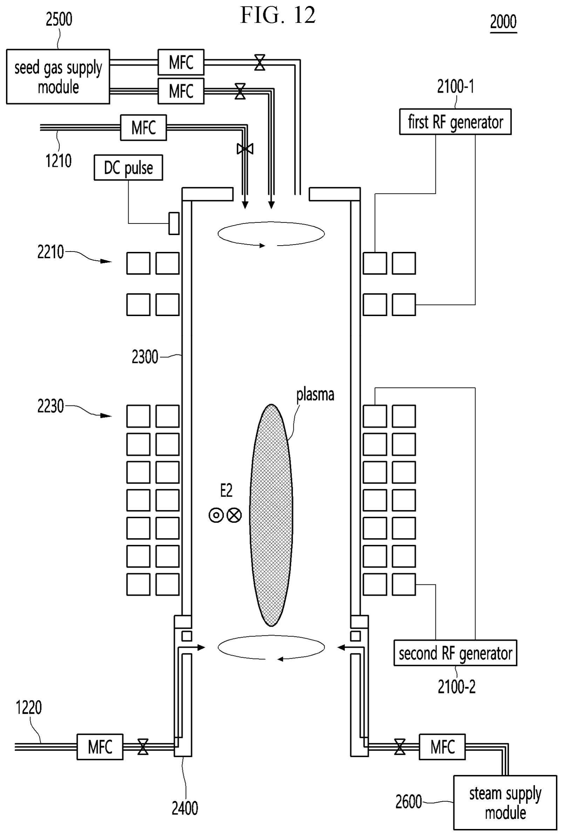

FIG. 11 and FIG. 12 are views illustrating a process in which plasma reforming proceeds according to an embodiment.

Referring to FIG. 10, the plasma reforming method may include: supplying a seed gas (S1100); applying power to the DC electrode and the auxiliary antenna module 2210 (S1200); applying power to the main antenna module 2230 (S1300); supplying a target gas to be reformed (S1400); stopping the supply of the seed gas or changing supplied seed gas (S1500); and controlling the flow rates of the supplied gases (S1600).

Each step will be described below.

The seed gas may be supplied to the discharge tube 2300 (S1100), and the power may be applied to the DC electrode and the auxiliary antenna module 2210 (S1200). Through steps S1100 and S1200, a plasma ignition process may be carried out.

Referring to FIG. 11, during the plasma ignition process, the seed gas is introduced into the discharge tube 2300, and when a DC pulse is applied to the DC electrode, a high voltage is applied into the discharge tube 2300, causing the seed gas to become ionized, thereby increasing the electron density, and When a voltage is applied to the auxiliary antenna module 2210 by the first RF generator 2100-1, an electric field E1 is formed. Accordingly, the introduced seed gas is then accelerated by the electric field E1 and undergoes a phase transition into a plasma state. During the plasma ignition process, the plasma transitions from an E-mode, in which capacitive coupling is dominant, to an H-mode, in which inductive coupling is dominant, as the electron density increases.

Subsequently, the power may be applied to the main antenna module 2230 (S1300). Step S1300 may be understood as a process of sustaining the ignited plasma.

Referring to FIG. 12, when a voltage is applied to the main antenna module 2230 by the second RF generator 2100-2, a continuously varying magnetic field is generated, inducing an electric field E2 inside the discharge tube 2300. And particles in the H-mode plasma state continuously move under the influence of the induced electric field E2, thereby allowing the plasma to be stably sustained. At this time, the plasma may move from an area corresponding to the auxiliary antenna module 2210 to an area corresponding to the main antenna module 2230 in the plasma induction region PIR.

As the plasma sustainment process proceeds, the target gas to be reformed may be supplied (S1500).

The target gas to be reformed may include the aforementioned first sub-feed stream, second sub-feed stream, and steam. The first sub-feed stream, second sub-feed stream, and steam may be supplied either simultaneously or sequentially. For example, the steam may be supplied first, followed by injecting the first sub-feed stream at a 1-1 flow rate and the second sub-feed stream at a 2-1 flow rate. Thereafter, the first sub-feed stream may be supplied at a 1-2 flow rate greater than the 1-1 flow rate, and the second sub-feed stream may be supplied at a 2-2 flow rate greater than the 2-1 flow rate (wherein the 1-2 flow rate may represent the maximum flow rate at which the first sub-feed stream can be supplied, and the 2-2 flow rate may represent the maximum flow rate at which the second sub-feed stream can be supplied). In another example, the first sub-feed stream may be supplied first, followed by simultaneous supplying the second sub-feed stream and the steam. In yet another example, the first sub-feed stream, the second sub-feed stream, and the steam may be supplied simultaneously. In still another example, the first sub-feed stream and the second sub-feed stream may be supplied simultaneously, and the steam may be supplied thereafter. In still another example, the steam may be supplied first as a gas for sustaining plasma discharge, and the first sub-feed stream and the second sub-feed stream may be supplied thereafter.

The supply or interruption of the supply of the first sub-feed stream may be performed by opening and closing a valve installed at an arbitrary point along the pipe through which the first sub-feed stream flows, and by a mass flow controller. The supply or interruption of the supply of the second sub-feed stream may be performed by opening and closing a valve installed at an arbitrary point along the pipe through which the second sub-feed stream flows, and by a mass flow controller. The supply or interruption of the supply of the steam may be performed by opening and closing a valve installed at an arbitrary point along the pipe through which the steam flows, and by a mass flow controller.

Meanwhile, the feed stream introduced through the first inlet 2310 and the feed stream introduced through the second inlet 2320 may have opposite swirl directions. For example, when the swirl direction of the first sub-feed stream introduced through the first inlet 2310 is counterclockwise (or clockwise) with respect to the central axis of the discharge tube 2300, the swirl direction of the second sub-feed stream introduced through the second inlet 2320 may be clockwise (or counterclockwise). In the case of the steam, it may be supplied in either the same swirl direction or the opposite swirl direction as that of the second sub-feed stream. As such, when the swirl directions of the gases supplied through the first inlet 2310 and the second inlet 2320 differ, they may collide with each other to create turbulence, which may increase the gas reaction rate.

The supply of the seed gas may be stopped or the supplied seed gas may be changed (S1400). This is because seed gases such as argon have properties that make it difficult to separate when mixed with syngas, so the seed gas should preferably not be supplied during syngas generation. In other words, argon may not be supplied during syngas generation. Alternatively, a seed gas that is easy to separate from the syngas or does not require separation (e.g., hydrogen gas) may be used as the seed gas during syngas generation. Alternatively, a gas that is reformed into a syngas (e.g., methane or carbon dioxide) may be used as the seed gas during syngas generation.

The supply or interruption of the supply of the seed gas may be performed by opening and closing a valve installed at an arbitrary point along the pipe through which the seed gas flows, and by a mass flow controller.

The time at which the supply of the seed gas is stopped (or the seed gas is replaced from argon to another gas) may be either when a voltage measured at the sub-antenna module 2210 exceeds a critical range (wherein the critical range corresponds to a criterion for plasma ignition and sustainment), or when a predetermined time elapses thereafter.

The target gas to be reformed may be supplied from a predetermined time before the time at which the supply of the seed gas is stopped. More specifically, the flow rate of the seed gas may begin to decrease at the time the target gas to be reformed is supplied or after a predetermined time thereafter. Here, the predetermined time may refer to the time required for the plasma to reach a steady state. In another example, the target gas to be reformed may be supplied at the time when the supply of the seed gas is stopped. In yet another example, the target gas to be reformed may be supplied from a predetermined time after the time at which the supply of the seed gas is stopped.

The supply of the seed gas may be gradually reduced before it is stopped. For example, the flow rate of the seed gas may be stepwise reduced by a predetermined amount from a predetermined time after the power is applied to the main antenna module 2230, from the time at which the target gas to be reformed is supplied, or from a predetermined time thereafter. More specifically, the flow rate of the seed gas may be reduced by a first amount at a first time after the target gas to be reformed is supplied, and further reduced by a second amount at a second time after the first time. In this case, it may be determined whether a plasma sustainment condition is satisfied after the first time. The plasma sustainment condition may refer to the condition in which power consumption of the plasma load is maintained. When it is determined that the plasma sustainment condition is satisfied, the flow rate of the seed gas may be reduced by the second amount at the second time, and after the second time, the determination of whether the plasma sustainment condition is satisfied may be performed again. Meanwhile, the flow rate of the seed gas may be reduced by the second amount after a predetermined time elapses following the first time, regardless of whether the plasma sustainment condition is satisfied.

The time at which the supply of the seed gas is stopped may vary depending on the type of final product. For example, when the final product is high-purity hydrogen and a seed gas such as argon is contained in the syngas, separation of hydrogen and argon gas in the syngas at the post-treatment unit 3000 is relatively difficult. Therefore, it is necessary to prevent the seed gas from being contained in the syngas. Therefore, for the final product being high-purity hydrogen, the supply of the seed gas may be stopped before the target gas to be reformed is supplied. Alternatively, the supply of the seed gas may be stopped after the target gas to be reformed is supplied, but until the seed gas supply is stopped, the reformed gas may not be provided to the post-treatment unit 3000 and may instead be discharged externally.

The flow rates of the supplied gases may be controlled during the plasma reforming process (S1600).

The structure and configuration of the post-treatment unit 3000 may vary depending on the type of final product to be obtained through the gas reforming system 100. And the gas composition ratio within the syngas supplied to the post-treatment unit 3000 may be determined differently depending on the post-treatment unit 3000. For example, when the final product is high-purity hydrogen, a higher hydrogen ratio in the syngas is preferred regardless of the post-treatment unit 3000. In another example, when the final product is methanol, the post-treatment unit 3000 may include a configuration that facilitates hydrogenation of carbon dioxide or carbon monoxide, and the gas composition ratio in the syngas supplied to the post-treatment unit 3000 is preferably such that the hydrogen-to-carbon monoxide ratio (H₂/CO) is preferably about 2.6 and/or the H₂/(2CO+3CO₂) ratio is about 1.05. In yet another example, when the final product is aviation fuel, the post-treatment unit 3000 may perform a Fischer-Tropsch (FT) process, and the syngas supplied to the post-treatment unit 3000 preferably has a hydrogen-to-carbon monoxide ratio (H₂/CO) of about 1.8 to about 2.2 and a relatively low amount of steam.

In other words, the composition ratio of the syngas generated through plasma reforming needs to be controlled within a specific range considering the post-treatment process. To this end, the ratio of gases supplied to the plasma induction region PIR may be adjusted. Specifically, the flow rates of the first sub-feed stream, the second sub-feed stream, and the steam supplied to the plasma induction region PIR may be controlled.

For example, when the final product is high-purity hydrogen, the gas composition ratio inside the discharge tube 2300 needs to be controlled to satisfy, as closely as possible, a composition ratio condition of methane: carbon dioxide:steam=about 1:0.5:2.

In another example, when the final product is methanol, the gas composition ratio inside the discharge tube 2300 needs to be controlled to satisfy, as closely as possible, a composition ratio condition of methane: carbon dioxide:steam=about 1:0.35:1.6.

In yet another example, when the final product is synthetic crude oil (or aviation fuel), the gas composition ratio inside the discharge tube 2300 needs to be controlled to satisfy, as closely as possible, a composition ratio condition of methane:carbon dioxide:steam=about 1:0.7:1.5.

The central control unit of the gas reforming system 100 or the controller of the plasma reformer 2000 may control the flow rates of the first sub-feed stream, the second sub-feed stream, and the steam such that the gas composition ratio inside the discharge tube 2300 satisfies the aforementioned composition ratio conditions, based on sensor values obtained by measuring the gas flow rates.

As described above, the flow rates of the gases may be controlled by the central control unit of the gas reforming system 100 or the controller of the plasma reforming unit 2000. At this time, sensors that detect the gas composition ratio or specific gas component ratio in the first sub-feed stream and the second sub-feed stream may be utilized. Specifically, a first gas sensor that measures the concentration of at least one of carbon dioxide and methane may be installed at an arbitrary point along the pipe through which the first sub-feed stream flows (e.g., first gas separation pipe 1210). Similarly, a second gas sensor that measures the concentration of at least one of carbon dioxide and methane may be installed at an arbitrary point along the pipe through which the second sub-feed stream flows (e.g., second gas separation pipe 1220). In addition, a third gas sensor that measures the concentration of steam may be installed at an arbitrary point along the pipe through which the steam flows. The central control unit or the controller of the plasma reforming unit 2000 may change the flow rate of at least one of the first sub-feed stream, the second sub-feed stream, and the steam by utilizing at least one of the first gas sensor, the second gas sensor, and the third gas sensor.

The syngas generated through reforming in the plasma induced region PIR of the discharge tube 2300 may flow to a lower portion of the discharge tube 2300. The syngas that has flowed to the lower portion of the discharge tube 2300 may undergo an additional reaction by the additional reaction module 2700, as will be described below.

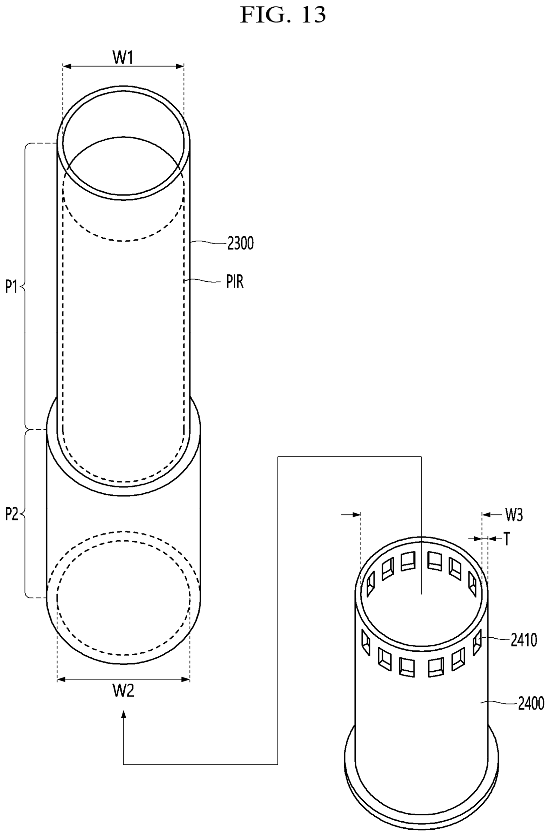

Hereinafter, a structure for supplying gases to below the plasma induction region PIR of the discharge tube 2300 will be described with reference to FIG. 13.

FIG. 13 is a view illustrating a discharge tube 2300 and a guide structure 2400 according to an embodiment.

Referring to FIG. 13, the discharge tube 2300 and the guide structure 2400 may be configured to be coupled to each other. For example, the discharge tube 2300 may be divided into a first portion P1 and a second portion P2, and the guide structure 2400 may be inserted into the second portion P2 of the discharge tube 2300.

The first portion P1 of the discharge tube 2300 may be understood as a part that defines a space in which plasma is induced. In addition, the first portion P1 may be understood as a part where the antenna structure 2200 is disposed. Furthermore, the first portion P1 may be understood as a part that defines a plasma induction region PIR.

The first portion P1 may include the aforementioned first inlet 2310.

The first portion P1 may be connected to the first gas separation pipe 1210.

The second portion P2 of the discharge tube 2300 may be understood as a part that defines a space behind a downstream end of the plasma induction region PIR. In addition, the second portion P2 may be understood as a part that defines a space into which the guide structure 2400 is inserted.

The second portion P2 may include the aforementioned second inlet 2320.

The second portion P2 may be connected to the second gas separation pipe 1220. Specifically, a gas supplied from the second gas separation pipe 1220 may flow to the second portion P2, or flow to the first portion P1 via the second portion P2.

Meanwhile, the antenna structure 2200 may be disposed only around the first portion P1 of the discharge tube 2300 and may not be disposed around the second portion P2 of the discharge tube 2300.

Alternatively, a portion of the antenna structure 2200 may be disposed around the first portion P1 of the discharge tube 2300, and another portion thereof may be disposed around the second portion P2 of the discharge tube 2300. In this case, more than half of the total number of layer antennas included in the antenna structure 2200 may be disposed around the first portion P1 of the discharge tube 2300. Alternatively, 80% or more of the total number of layer antennas included in the antenna structure 2200 may be disposed around the first portion P1 of the discharge tube 2300.

The first portion P1 of the discharge tube 2300 may have a first width W1 (or first diameter, first span). The second portion P2 of the discharge tube 2300 may have a second width W2 (or second diameter, second span). The first width W1 of the first portion P1 may be smaller than the second width W2 of the second portion P2. This is to prevent a reduction in the size of the flow path for a syngas due to the insertion of the guide structure 2400 into the second portion P2.

The guide structure 2400 may include an inner wall, an outer wall, and at least one hole 2410 formed through the inner and outer walls. As will be described below, a gas supplied to the discharge tube 2300 may flow along the outer wall of the guide structure 2400. The gas that has flowed along the outer wall of the guide structure 2400 may flow into the guide structure 2400 through the hole 2410.

The guide structure 2400 may be inserted into the second portion P2 of the discharge tube 2300. When the guide structure 2400 is inserted into the second portion P2, a gap may be formed between the guide structure 2400 and the second portion P2. Specifically, a gap may be formed between the outer wall of the guide structure 2400 and the inner wall of the second portion P2, and gases such as the second sub-feed stream and the steam may flow through the formed gap.

The gases that have flowed through the gap formed between the guide structure 2400 and the second portion P2 of the discharge tube 2300 may flow into the guide structure 2400 or the discharge tube 2300 through the hole 2410. In this case, the position of the hole 2410 may be located closer to an upper end than a lower end of the guide structure 2400. This is because when the guide structure 2400 is coupled to the second portion P2, the upper end of the guide structure 2400 is positioned closer to the plasma induced region PIR or the first portion P1 than the lower end, and the guide structure 2400 is configured to supply the gases to the plasma induced region PIR.

The guide structure 2400 may have a third width W3 (or third diameter, third span) and be designed to have a predetermined thickness T. In this case, the third width W3 may be designed to satisfy a first design condition in which it is equal to the first width W1 of the first portion P1 of the discharge tube 2300. In addition, the thickness T of the guide structure 2410 may be designed to satisfy a second design condition in which it is smaller than half the difference between the first width W1 of the first portion P1 of the discharge tube 2300 and the second width W2 of the second portion P2 of the discharge tube 2300. Accordingly, even when the guide structure 2410 is coupled to the discharge tube 2300, the flow path of the syngas generated through plasma reforming (since the first design condition is satisfied) may not become smaller, and a gap for gas flow may be formed between the discharge tube 2300 and the guide structure 2400 (since the second design condition is satisfied).

By utilizing the guide structure 2400, the second sub-feed stream and the steam may be introduced at a location relatively far from the plasma induction region PIR and flow to the downstream end of the plasma induction region PIR. In this case, since a gas inlet or gas flow pipe, which may be susceptible to thermal damage due to the high temperature of the plasma (about 800°C), is positioned away from the plasma, thermal damage may be prevented. Here, at least a portion of the guide structure 2400 may be made of a heat-resistant material, such as at least one of aluminum oxide, silicon nitride, silicon nitride, silicon dioxide, yttrium oxide, ceramic, silicon carbide, and a combination thereof.

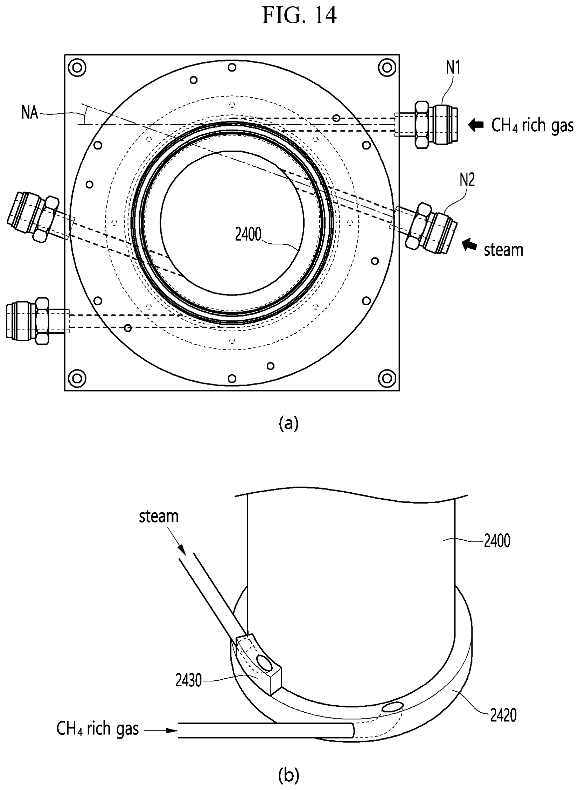

Hereinafter, a method and structure for supplying the second sub-feed stream and the steam will be described with reference to FIGS. 14A and 14B.

FIG. 14 is a view illustrating a first gas supply nozzle N1 and a second gas supply nozzle N2 according to an embodiment.

Referring to FIG. 14A, the first gas supply nozzle N1 may be understood as a nozzle through which the second sub-feed stream is supplied, and the second gas supply nozzle N2 may be understood as a nozzle through which the steam is supplied. Accordingly, the first gas supply nozzle N1 may be connected to the second gas separation pipe 1220, and the second gas supply nozzle N2 may be connected to the steam supply module 2600.

Specifically, the first gas supply nozzle N1 and the second gas supply nozzle N2 may be designed such that a gas supply direction is tangential to the outer wall of the guide structure 2400. Accordingly, the gases injected from the first gas supply nozzle N1 and the second gas supply nozzle N2 may flow along the outer wall of the guide structure 2400.

The first gas supply nozzle N1 and the second gas supply nozzle N2 may be arranged such that the gases are supplied in the same direction with respect to the central axis of the guide structure 2400. For example, the first gas supply nozzle N1 and the second gas supply nozzle N2 may be arranged to supply the gases in a clockwise or counterclockwise direction with respect to the central axis of the guide structure 2400.

Here, the first gas supply nozzle N1 and the second gas supply nozzle N2 may be arranged to have a predetermined angle. For example, the imaginary central axis of the first gas supply nozzle N1 and the imaginary central axis of the second gas supply nozzle N2 may form a nozzle angle NA.

The nozzle angle NA may be designed be closer to 0° or 180°, rather than 90°.

Each of the first gas supply nozzle N1 and the second gas supply nozzle N2 may be disposed at a predetermined angle with respect to the direction parallel to the central axis of the guide structure 2400. This is to ensure that the first and second gas supply nozzles N1 and N2 inject the gases from a lower portion of the guide structure 2400 and the injected gases flow more quickly toward the upper portion of a guide structure 2400.

The first gas supply nozzle N1 and the second gas supply nozzle N2 may be connected to stepped portions of the guide structure 2400. For example, referring to FIG. 14B, the guide structure 2400 may include a first stepped portion 2420 and a second stepped portion 2430. In this case, the first gas supply nozzle N1 may be connected to the first stepped portion 2420, and the second gas supply nozzle N2 may be connected to the second stepped portion 2430. Each of the stepped portions of the guide structure 2400 may include a nozzle coupling portion, a flow path, and a gas inlet hole. Each of the gas supply nozzles may be coupled to the nozzle coupling portion of the stepped portion, allowing the gas supplied from the gas supply nozzle to be introduced through the flow path and the gas inlet hole into a space between the outer wall of the guide structure 2400 and the inner wall of the discharge tube 2300.

The first stepped portion 2420 and the second stepped portion 2430 may have different heights with respect to the central axis of the guide structure 2400. For example, referring to FIG. 14B, the first stepped portion 2420 may be positioned lower than the second stepped portion 2430. This is to ensure that the first gas supply nozzle N1 and the second gas supply nozzle N2 supply the gases to the outer wall of the guide structure 2400 at different heights.

Meanwhile, both the first gas supply nozzle N1 and the second gas supply nozzle N2 may be connected to the first stepped portion 2420 or to the second stepped portion 2430. Alternatively, the first gas supply nozzle N1 may be connected to the second stepped portion 2430, and the second gas supply nozzle N2 may be connected to the first stepped portion 2420.

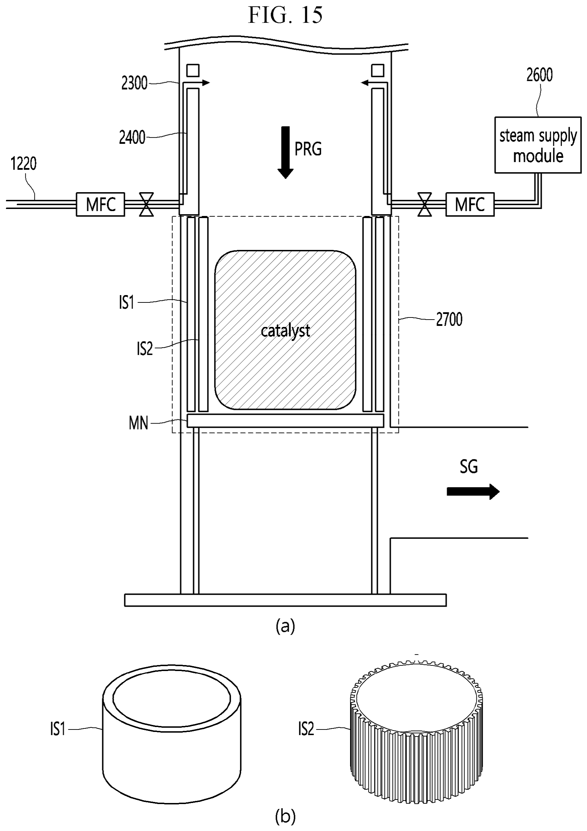

Hereinafter, the additional reaction module 2700 of the plasma reforming unit 2000 and the additional reaction performed in the additional reaction module 2700 will be described with reference to FIGS. 15A and 15B.

FIG. 15A is a view illustrating an additional reaction module 2700 according to an embodiment.

FIG. 15B is a view illustrating insulating structures of the additional reaction module 2700 according to an embodiment.

The additional reaction module 2700 may be understood as a configuration for improving the gas conversion efficiency within the plasma reforming unit 2000. In other words, the additional reaction module 2700 is a configuration for additionally reacting a gas that remains unreacted by plasma reforming.

Referring to FIG. 15A, the additional reaction module 2700 may include a mesh filter MN, a catalyst, a first insulating structure IS1, and a second insulating structure IS2.

The additional reaction module 2700 may be disposed below the discharge tube 2300 or the guide structure 2400. In other words, the additional reaction module 2700 may be located in a region through which a plasma-reformed gas PRG has passed through plasma flows before reaching the post-treatment unit 3000.

The additional reaction module 2700 may basically be understood as a configuration for inducing a catalytic reaction. Accordingly, the additional reaction module 2700 may include the catalyst and the mesh filter MN on which the catalyst is placed. Specifically, the mesh filter MN may be disposed at a predetermined position by a support, and a catalyst may be placed on top of the mesh filter MN.

Meanwhile, the catalytic reaction requires a high-temperature condition in which the catalyst is activated. The high-temperature condition for the catalytic reaction may be achieved by utilizing waste heat (or residual heat) from the plasma. In this case, the mesh filter MN may be made of a material resistant to corrosion and high temperatures, so as to prevent damage under high-temperature environment for the catalytic reaction or from the catalyst itself. For example, the mesh filter MN may be made of a material such as Inconel, stainless steel, iron-chromium, nickel, or Monel.

In addition, to satisfy the high-temperature condition required for the catalytic reaction, the first insulating structure IS1 and the second insulating structure IS2 may be employed. The first insulating structure IS1 and the second insulating structure IS2 are configured to block external heat from affecting the catalyst and to provide double insulation for the catalyst portion.

Referring to FIG. 15A, the second insulating structure IS2 may be disposed to surround the catalyst, and the first insulating structure IS1 may be disposed to surround the second insulating structure IS2.

Each of the first insulating structure IS1 and the second insulating structure IS2 may be at least partially made of a heat-resistant material, such as at least one of aluminum oxide, silicon nitride, silicon nitride, silicon dioxide, yttrium oxide, ceramic, silicon carbide, or a combination thereof.

Referring to FIG. 15B, the first insulating structure IS1 and the second insulating structure IS2 may have a cylindrical shape. In addition, the second insulating structure IS2 may have multiple grooves formed on an outer surface thereof. Accordingly, as illustrated in FIG. 15A, a gap may be formed between the first insulating structure IS1 and the second insulating structure IS2, thereby enhancing thermal insulation performance.

Meanwhile, the additional reaction module 2700 may be omitted.

The gas that has passed through the additional reaction module 2700 may flow to the post-treatment unit 3000. For convenience of explanation, the gas flowing to the post-treatment unit 300 will hereinafter be referred to as a syngas SG.

### [Post-treatment unit]

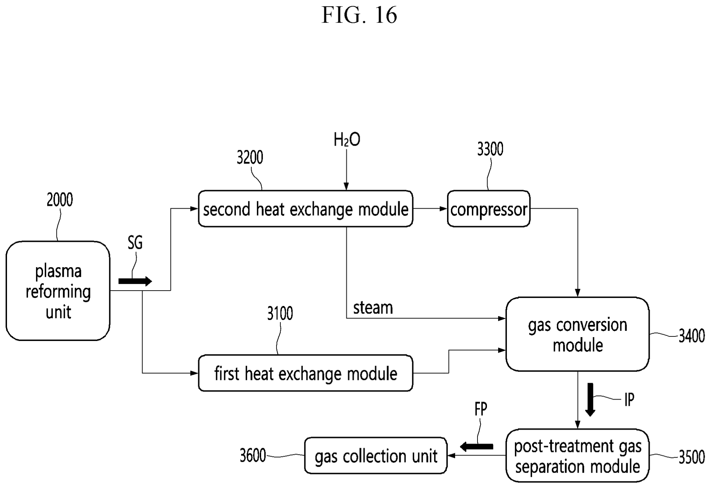

Hereinafter, the post-treatment unit 3000 will be described with reference to FIGS. 16 to 21.

### 1. Configuration of post-treatment unit

FIG. 16 is a view illustrating a plasma reforming unit 2000 and a post-treatment unit 3000 according to an embodiment.

Referring to FIG. 16, the post-treatment unit 3000 may include a first heat exchange module 3100, a second heat exchange module 3200, a compressor 3300, a gas conversion module 3400, a post-treatment gas separation module 3500, and a gas collection unit 3600.

A post-treatment process performed in the post-treatment unit 3000 may be broadly understood as including a gas conversion process and a gas separation process. The main components of these process are the gas conversion module 3400 and the post-treatment gas separation module 3500.

First, a syngas SG generated in the plasma reforming unit 2000 may flow to the gas conversion module 3400 and be subjected to a reaction where hydrogen or carbon monoxide in the syngas is converted into specific gases. At this time, the configuration or structure of the gas conversion module 3400 may vary depending on the type of final product to be produced by the gas reforming system 100, and the types of reactions to be induced may also differ.

For example, the gas conversion module 3400 may be a water gas shift (WGS) module. The WGS module may perform a process of converting carbon monoxide in the syngas into hydrogen through a catalytic reaction. Specifically, the WGS module may receive the syngas and steam and induce the following reaction:

CO+H₂O->CO₂+H₂

In the case of the water gas shift module, the conditions for the water gas shift reaction are as follows: i) the internal temperature needs to be about 180°C to 250°C or about 300°C to 450°C; ii) there are no particular limitations on internal pressure the internal pressure, it needs to be preferably about 10 bar; and iii) the ratio of hydrogen to carbon monoxide (H₂/CO) in the supplied gas needs to be about 3 to 10. However, the conditions for the water gas shift reaction are not limited to those described above.

In the water gas shift module, the catalyst may be an oxide of a metal such as iron, nickel, or chromium, and a reducing agent needs to be added to activate the catalyst.

When the final product to be produced in the gas reforming system 100 is high-purity hydrogen, the water gas shift module may be selected as the gas conversion module 3400. Alternatively, the water gas shift module may be selected as the gas conversion module 3400 in the process of collecting carbon dioxide through the gas reforming system 100.

In another example, the gas conversion module 3400 may be a Fischer-Tropsch process module. The Fischer-Tropsch process module may perform a process of synthesizing liquid hydrocarbons from the syngas through a catalytic reaction. Specifically, the Fischer-Tropsch process module receive the syngas and induce the following reaction:

(2n+1) H₂+ (n) CO->C₍ₙ₎H₍₂ₙ₊₂₎+ (n) H₂O

In the case of the Fischer-Tropsch process module, the conditions for the aforementioned reaction are as follows: i) the internal temperature needs to be about 220°C to 230°C (when the catalyst is cobalt) or about 300°C to 320°C (when the catalyst is iron); ii) the internal pressure needs to be about 20 bar (when the catalyst is either cobalt or iron); and iii) the ratio of hydrogen to carbon monoxide (H₂/CO) in the supplied gas needs to be about 2.0 to 2.1.

However, the conditions for the Fischer-Tropsch reaction are not limited to those described above.

In the Fischer-Tropsch process module, the catalyst may be an oxide of a metal such as iron, cobalt, ruthenium, nickel, or chromium, and a reducing agent needs to be added to activate the catalyst.

When the final product to be produced in the gas reforming system 100 is aviation fuel, the Fischer-Tropsch process module may be selected as the gas conversion module 3400.

In another example, the gas conversion module 3400 may be a methanol synthesis module. The methanol synthesis module may be implemented as a fixed bed reactor or a fluidized bed reactor, and may perform a process of synthesizing methanol from the syngas through a catalytic reaction.

In the case of the methanol synthesis module, the conditions for the methanol synthesis are as follows: i) the internal temperature needs to be about 240°C to 260°C; ii) the internal pressure needs to be about 50 to 80 bar; and iii) the ratio of hydrogen to carbon monoxide (H₂/CO) in the supplied gas needs to be about 2.6.

However, the conditions for the methanol synthesis are not limited to those described above.

In the methanol synthesis module, the catalyst may be an oxide of zinc, chromium, copper-zinc, or copper-manganese, and a reducing agent needs to be added to activate the catalyst.

When the final product to be produced in the gas reforming system 100 is methanol, the methanol synthesis module may be selected as the gas conversion module 3400.

Meanwhile, the gas conversion module 3400 may be configured as other process modules besides the aforementioned ones, and may also be composed of multiple process modules.

The gas discharged through the gas conversion module 3400 may be supplied to the post-treatment gas separation module 3500. For convenience of explanation, the gas discharged from the gas conversion module 3400 will hereinafter be referred to as intermediate product (IP).

The post-treatment gas separation module 3500 may perform a gas separation process. The gas separation process performed in the post-treatment gas separation module 3500 may be understood as a process for increasing the yield efficiency of the final product.

The post-treatment gas separation module 3500 may be implemented using a pressure swing adsorption (PSA) method. The PSA method separates gases by using an adsorbent to preferentially adsorb components with high selectivity from a passing gas, thereby allowing components with lower selectivity to be discharged first.

The post-treatment gas separation module 3500 may be implemented in a form that includes a separation membrane, similar to the aforementioned pre-treatment gas separation module 1200.

The gas discharged from the post-treatment gas separation module 3500 may be collected as a final product FP in the gas collection unit 3600. Additional configurations for varying pressure or temperature may also be provided between the post-treatment gas separation module 3500 and the gas collection unit 3600.

The first heat exchange module 3100 and the second heat exchange module 3200 may function to controlling the temperature of the gas passing therethrough.

The temperature of plasma induced in the plasma reforming unit 2000 ranges from about 800°C to 2000°C, and accordingly, the temperature of the syngas SG may also reach several hundred to 2,000°C. As described above, since the temperature condition for gas conversion is relatively low, it is necessary to cool the syngas SG to lower its temperature.

In other words, the first heat exchange module 3100 and the second heat exchange module 3200 may cool the syngas passing therethrough such that the temperature of the syngas reaches a required temperature for the gas conversion process.

The second heat exchange module 3200 may receive water and discharge steam. Specifically, the second heat exchange module 3200 may cool the supplied syngas SG, and transfer its thermal energy to water, thereby generating and discharging steam. The steam discharged from the second heat exchange module 3200 may flow to the gas conversion module 3400.

The first heat exchange module 3100 and the second heat exchange module 3200 may be implemented in various forms, such as a surface heat exchanger such as a multi-pipe heat exchanger or a double-pipe heat exchanger, a regenerative heat exchanger such as a rotary regenerative heat exchanger or a valve-type annular regenerative heat exchanger, a liquid-connected indirect heat exchanger, or a direct contact heat exchanger.

The compressor 3300 may increase the pressure of the gas passing therethrough. The syngas SG discharged from the plasma reforming unit 2000 may have atmospheric pressure or higher. Since the pressure condition for gas conversion is relatively higher than atmospheric pressure as described above, the syngas SG needs to be pressurized before being supplied to the gas conversion module 3400.

The compressor 3300 may increase the pressure of the supplied syngas SG.

The compressor 3300 may be implemented as a reciprocating compressor, a rotary compressor, a screw compressor, or a centrifugal compressor.

### 2. Path from plasma reforming unit to gas conversion module

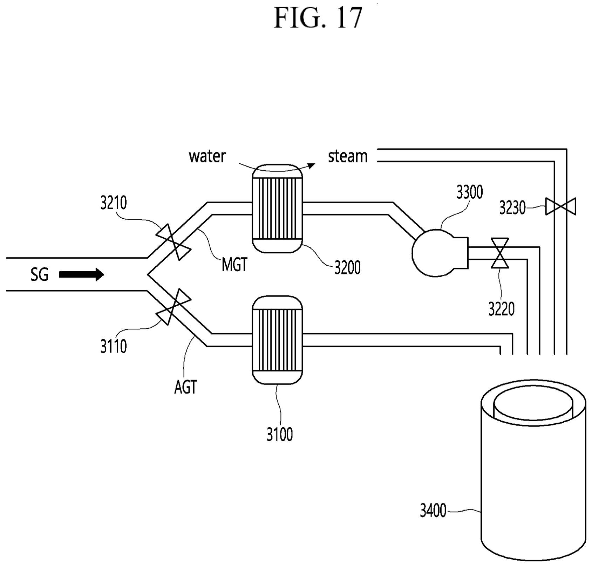

Meanwhile, the syngas SG discharged from the plasma reforming unit 2000 may reach the gas conversion module 3400 through at least two paths. Hereinafter, the path through which the syngas SG flows to the gas conversion module 3400 will be described with reference to FIG. 17.

FIG. 17 is a view illustrating a structure for allowing a syngas SG to flow to a gas conversion module 3400 according to an embodiment. Referring to FIG. 17, the post-treatment unit 3000 may include a first valve 3110, a second valve 3210, a third valve 3220, and a fourth valve 3230 to control the flow of gases. The first to fourth valves 3110, 3210, 3220, and 3230 may be used to determine the timing of fluid flow within pipes where they are installed, and at least one of these valves may be omitted.

Referring to FIG. 17, the first valve 3110 may be installed between a discharge pipe of the plasma reforming unit 2000 and the first heat exchange module 3100, the second valve 3210 may be installed between a discharge pipe of the plasma reforming unit 2000 and the second heat exchange module 3200, the valve 3220 may be installed between the compressor 3300 and the gas conversion module 3400, and the fourth valve 3230 may be installed at an arbitrary point along a steam inlet pipe.

Referring to FIG. 17, the syngas SG may flow to the gas conversion module 3400 via at least a first path passing through an auxiliary gas pipe AGT and the first heat exchange module 3100. In addition, the syngas SG may flow to the gas conversion module 3400 via at least a second path passing through a main gas pipe MGT, the second heat exchange module 3200, and the compressor 3300.

The first path may be understood as a route for creating a process preparation environment within the gas conversion module 3400, as will be described below. For example, at the initial stage of the gas conversion process, the syngas SG may flow to the gas conversion module 3400 through the first path.

The flow of the syngas SG to the first path may be controlled by an opening and closing operation of the first valve 3110.

The second path, as will be described below, may be understood as a route for supplying the gas to proceed the process within the gas conversion module 3400. For example, the syngas SG may flow to the gas conversion module 3400 through the second path when a process preparation environment is created within the gas conversion module 3400.

The flow of the syngas SG to the second path may be controlled by an opening and closing operation of the second valve 3210.

The syngas SG may flow to either the first path or the second path. Alternatively, the syngas SG may flow to the first path while simultaneously flowing to the second path, and vice versa. Hereinafter, for convenience of explanation, the syngas SG flowing to the first path will be referred to as a first path stream, and the syngas SG flowing to the second path will be referred to as a second path stream. However, it should be understood that these may also be referred to by other terms such as first syngas and second syngas. Depending on the gas flow control within the post-treatment unit 3000, the entirety of the syngas SG may form either the first path stream or the second path stream. Alternatively, the syngas SG may be divided into both the first path stream and the second path stream.

The first path stream supplied to the gas conversion module 3400 through the first path may have a lower pressure than the second path stream supplied to the gas conversion module 3400 through the second path.

### 3. Gas conversion method

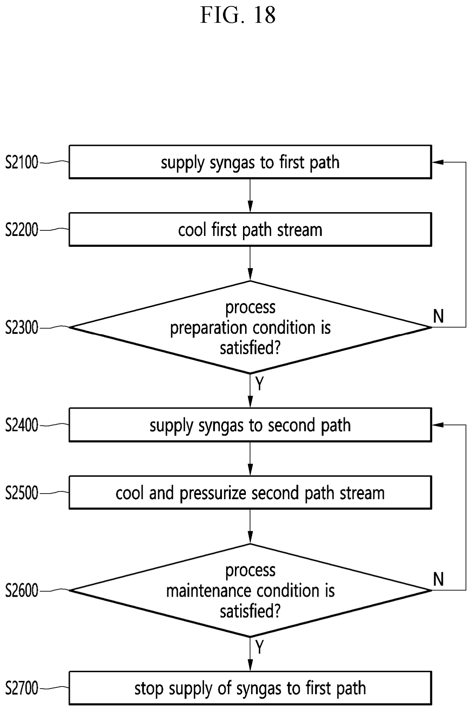

Hereinafter, a gas conversion method will be described with reference to FIGS. 18 to 22.

FIG. 18 is a view illustrating a gas conversion method according to an embodiment.

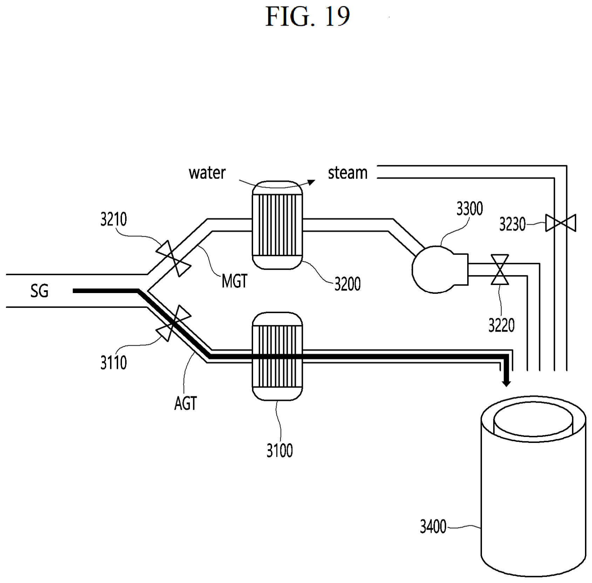

FIGS. 19 to 21 are views illustrating a process in which gas conversion proceeds according to an embodiment.

Referring to FIG. 18, the gas conversion method may include: supplying a syngas SG to a first path (S2100); cooling a first path stream (S2200); determining whether a process preparation condition is satisfied (S2300); supplying the syngas SG to a second path (S2400); cooling and pressurizing a second path stream (S2500); determining whether a process maintenance condition is satisfied (S2600); and stopping the supply of the syngas SG to the first path (S2700).

Each step will be described in detail below.

As described above, in order for most types of gas conversion modules 3400 to operate, the interiors thereof need to reach a specific temperature, and a reducing agent needs to be supplied to activate a metal oxide catalyst. Therefore, before supplying a target gas to be converted, a preparatory process for pre-heating the gas conversion module 3400 and activating the catalyst needs to be carried out.

Steps S2100, S2200, and S2300 may be understood as steps for process preparation. Furthermore, steps S2100, S2200, and S2300 may be understood as being performed within an initial reduction section (or first time section). Here, the initial reduction section may also be interpreted as a time section during which the syngas SG is supplied to the gas conversion module 3400 via the first path.

First, the syngas SG may be supplied to the first path S2100. At this time, the syngas SG transferred to the first path and passing through the first heat exchange module 3100 may be understood as the first path stream.

The central control unit or the controller of the post-treatment unit 3000 may control the first valve 3110 to supply the syngas SG to the first heat exchange module 3100. At this time, the opening time of the first valve 3110 may be within a predetermined time from the time at which power is applied to the aforementioned main antenna module 2230. Alternatively, the opening time of the first valve 3110 may be within a predetermined time from the time at which any one of the first sub-feed stream, the second sub-feed stream, and the steam begins to be supplied to the discharge tube 2300. Alternatively, the opening time of the first valve 3110 may be within a predetermined time from the time at which the concentration of the syngas SG within the discharge tube 2300 becomes equal to or greater than a predetermined value. Alternatively, the first valve 3110 may be in an open state even before plasma reforming begins.

Cooling of the first path stream may be performed in the first heat exchange module 3100 (S2200).

The first heat exchange module 3100 may cool the first path stream to a first temperature range. Here, the first temperature range may vary depending on the optimal reaction environment of the gas conversion module 3400. For example, when the gas conversion module 3400 is the aforementioned water gas shift module, the first heat exchange module 3100 may cool the first path stream to a temperature range of 150°C to 400°C. Alternatively, the first temperature range may be a temperature range required to activate the catalyst of the gas conversion module 3400. Alternatively, considering the length of the path from the first heat exchange module 3100 to the gas conversion module 3400, the first temperature range may be a temperature range higher than the optimal reaction temperature of the gas conversion module 3400.

Referring to FIG. 19, the syngas SG may be supplied to the first heat exchange module 3100, and the first path stream cooled in the first heat exchange module 3100 may flow to the gas conversion module 3400. In other words, the entirety of the syngas SG may flow to the gas conversion module 3400 through the first path, and no syngas SG may flow to the second path.

In FIG. 19, the first path stream flowing to the gas conversion module 3400 through the first path contains hydrogen, and the hydrogen may act as a reducing agent to activate the catalyst of the gas conversion module 3400. Additionally, since the temperature of the first path stream may be controlled to a level required for the catalytic reaction or catalyst activation, the supply of the first path stream through the first path may create the internal environment of the gas conversion module 3400 into one suitable for initiating the process.

As described above, when the process preparation environment for the gas conversion module 3400 is created using the syngas produced in the plasma reforming unit 2000, there is no need for separate equipment to pre-heat the gas conversion module 3400 or activate the catalyst. Accordingly, the construction cost of the gas reforming system 100 may be significantly reduced, and the reduction in components may also enhance ease of control within the system.

As the process preparation progresses, it may be determined whether the process preparation condition is satisfied (S2300). Here, the process preparation condition may be understood as a criterion for determining whether the process preparation has been completed and the gas conversion process can proceed. In other words, when the process preparation condition is satisfied, the gas conversion process may proceed in the gas conversion module 3400.

The process preparation condition may vary depending on the type of gas conversion module 3400. For example, when the gas conversion module 3400 is the water gas shift module, the process preparation condition may include the following conditions and combinations thereof: a first condition in which the internal temperature is maintained within a range of about 150°C and about 400°C; a second condition in which a predetermined proportion or more of the catalyst is activated; and a third condition in which a predetermined time elapses from the time when the syngas SG begins to be supplied to the first path. Here, a component analysis device may be used to determine whether the second condition is satisfied. The component analysis device may, for example, monitor the amount of hydrogen (e.g., hydrogen peak) within the gas conversion module 3400, and determine whether the catalyst is activated based on the degree of reduction in the amount of hydrogen.

When the process preparation condition is not satisfied, the syngas SG may continue to be supplied to the first heat exchange module 3100. This may indicate that the supply of the syngas SG to the first heat exchange module 3100 continues without interruption. On the one hand, it is also possible to supply the syngas SG to the first heat exchange module 3100 only for a predetermined period of time, and when the process preparation condition is not satisfied, supply the syngas SG to the first heat exchange module 3100 again only for another predetermined period of time.

When the process preparation condition is satisfied, the syngas SG may be supplied to the second path (S2400). The time section during which the syngas SG is supplied to the second path may be understood as a main reaction section (or second time section). At this time, the syngas SG transferred to the second path and passing through the second heat exchange module 3200 may be understood as the second path stream.

The central control unit or the controller of the post-treatment unit 3000 may control the second valve 3210 to supply the syngas SG to the second heat exchange module 3200. At this time, the opening time of the second valve 3210 may be after the aforementioned process preparation condition is satisfied.

Meanwhile, the opening time of the second valve 3210 may be before the closing time of the first valve 3110. For example, since the pressurization process at the compressor 3300 in the second path may require a predetermined time, the second valve 3210 may be opened before the first valve 3110 closes, while the third valve 3220 may be controlled to remain closed. This ensures that the second path stream is pressurized before the process preparation condition is satisfied, but remains unsupplied to the gas conversion module 3400. At this time, steam may also generated but controlled such that it is not supplied to the gas conversion module 3400 by the fourth valve 3230.

Meanwhile, when the fourth valve 3230 is open, at least one of the first valve 3110 and the third valve 3220 needs to be open. In other words, it is necessary to prevent the steam from being supplied alone to the gas conversion module 3400. This is because supplying the steam alone to the activated catalyst may lead to a deterioration in catalyst performance.

Cooling and pressurization of the second path stream may be performed (S2500). Specifically, the second path stream may be cooled in the second heat exchange module 3200 and pressurized in the compressor 3300.

The second heat exchange module 3200 may cool the supplied second path stream to a second temperature range. Here, the second temperature range may correspond to a room temperature range (about 15°C to 20°C). Before pressurization by the compressor 3300, the second path stream needs to have moisture removed therefrom using a dehumidification module (not illustrated), and therefore the room temperature range may be understood as a suitable temperature range for effectively removing moisture. The second temperature range may also be determined by further considering the length of the path from the second heat exchange module 3200 to the gas conversion module 3400.

The first temperature range in which the first path stream is cooled by the first heat exchange module 3100 may differ from the second temperature range in which the second path stream is cooled by the second heat exchange module 3200. For example, the minimum value of the first temperature range may be higher than the maximum value of the second temperature range.

The first temperature range in which the first path stream is cooled by the first heat exchange module 3100 may be the same as the second temperature range in which the second path stream is cooled by the second heat exchange module 3200.

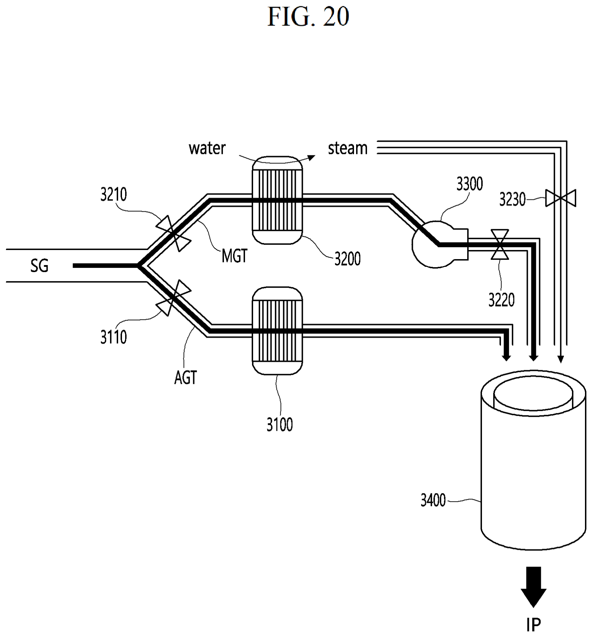

Referring to FIG. 20, the syngas SG may be supplied to the second heat exchange module 3200, and the second path stream cooled by the second heat exchange module 3200 may flow to the compressor 3300 to be pressurized and then flow to the gas conversion module 3400. The temperature of the second path stream before being supplied to the gas conversion module 3400 may be about 150°C, and for this purpose, a separate heating module may be provided. The flow of the fluid from the compressor 3300 to the gas conversion module 3400 may be controlled by an opening and closing operation of the third valve 3220.

When the second path stream flows to the gas conversion module 3400, the gas conversion process may proceed and the gas conversion module 3400 may discharge an intermediate product IP. The discharged intermediate product IP may be supplied to the aforementioned post-treatment gas separation module 3500.

At this time, as described above, water may receive thermal energy from the second heat exchange module 3200 and be converted into steam, which may be supplied to the gas conversion module 3400 through a separate pipe. Here, the steam may serve to maintain the internal temperature of the gas conversion module 3400 at the optimal reaction temperature. In addition, depending on the type of gas conversion module 3400, the steam may also be used to produce a target intermediate product IP by reacting with the supplied syngas (e.g., carbon monoxide or carbon dioxide). Depending on the type of gas conversion module 3400, the steam may not be supplied. The flow of the steam may be controlled by an opening and closing operation of the fourth valve 3230.

Meanwhile, referring to FIG. 20, when a portion of the syngas SG flows through the second path, another portion of the syngas SG may be continuously (or periodically) supplied to the gas conversion module 3400 through the first path. This is because, when the supply of the first path stream through the first path is immediately stopped at the time when the supply of the second path stream through the second path begins, the environment created for the catalytic reaction in the gas conversion module 3400 (e.g., temperature condition) may deteriorate, potentially resulting in a reduced catalytic reaction rate.

Therefore, as will be described below, the time at which the supply of the syngas SG through the first path is stopped needs to be precisely determined by considering the process maintenance condition.

According to the gas conversion method, it may be determined whether the process maintenance condition is satisfied (S2600). The process maintenance condition may refer to a criterion for maintaining the gas conversion process.

The process maintenance condition may vary depending on the type of gas conversion module 3400. For example, when the gas conversion module 3400 is the water gas shift module, the process maintenance condition may include the following conditions and combinations thereof: a first maintenance condition in which the internal temperature is maintained within a range of about 150°C and about 400°C for equal to or longer than a predetermined time; a second maintenance condition in which a predetermined proportion or more of the catalyst is activated; a third maintenance condition in which a predetermined time elapses from the time when the syngas SG begins to be supplied to the second path; and a fourth maintenance condition in which the amount of steam supplied to the gas conversion module 3400 becomes equal to or greater than a predetermined amount. Here, the aforementioned component analysis device may be used to determine whether the second maintenance condition is satisfied.

When the process maintenance condition is not satisfied, the syngas SG may be continuously supplied to the second heat exchange module 3200. At this time, the supply of the syngas SG through the first path may not be stopped. This may be understood to mean that the supply of the syngas SG through the first path needs to be maintained until the process maintenance condition is satisfied.

When the process maintenance condition is satisfied, the supply of the syngas SG to the first heat exchange module 3100 may be stopped (S2700). The central control unit or the controller of the post-treatment unit 3000 may control the first valve 3110 to stop the flow of the syngas SG through the first path.

At this time, the closing time of the first valve 3110 may be after the aforementioned process maintenance condition is satisfied.

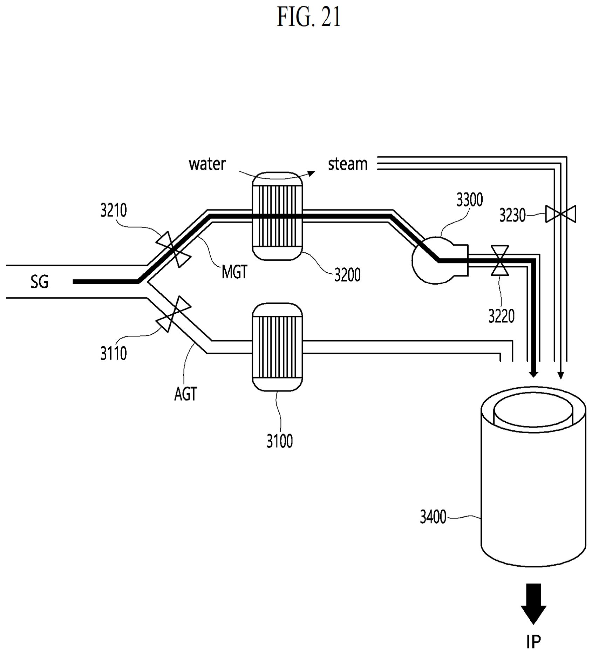

Referring to FIG. 21, the entirety of the syngas SG may flow to the gas conversion module 3400 through the second path, and the steam may flow to the gas conversion module 3400 through another path, while no syngas SG may flow to the first path.

As described above, the gas conversion method may be divided into a process preparation process (step S2100, step S2200, and step S2300) and a process progress process (step S2400, step S2500, step S2600, and step S2700).

Here, the syngas reformed in the plasma reforming unit 2000 may be utilized in both the preparation of the gas conversion process and the gas conversion process itself. However, when the start of the gas conversion process is excessively delayed or the process preparation continues unnecessarily, a situation may arise in which the syngas to be converted is wasted in the process preparation. Therefore, in that the syngas SG is supplied through the second path as quickly as possible once the process preparation condition is satisfied, and that the supply of the syngas SG through the first path is stopped as quickly as possible once the process maintenance condition is satisfied, steps S2300 and S2600 may be understood as critical steps to prevent unnecessary waste of the syngas and, furthermore, to prevent a decrease in the efficiency of the gas conversion process (or the overall efficiency of the gas reforming system 100).

The features, structures, effects, etc. described in the embodiments are included in at least one embodiment of the present disclosure and are not necessarily limited to one embodiment. Furthermore, the features, structures, effects, etc. provided in each embodiment can be combined or modified in other embodiments by those skilled in the art to which the embodiments belong. Therefore, the contents related to the combination and modification should be construed to be included in the scope of the present disclosure.

In addition, the embodiments described herein have been provided merely for illustrative purposes, those skilled in the art will appreciate that various modifications, additions, and substitutions are possible, without departing from the scope and spirit of the present disclosure. In other words, each element specifically shown in the embodiments can be implemented in modified forms. Therefore, the differences related to the modification and application should be construed to be included in the scope of the present disclosure as disclosed in the accompanying claims.

## Claims

1. A method for reforming gas comprising:
① forming a first sub-feed stream and a second sub-feed stream by supplying a desulfurized feed gas to a first gas separation module,
wherein the first gas separation module comprising a porous separation membrane,
wherein the first sub-feed stream comprises the feed gases that do not pass through the porous separation membrane, wherein the second sub-feed stream comprises the feed gases that pass through the porous separation membrane,
wherein a molar ratio of carbon dioxide in the first sub-feed stream is higher than a molar ratio of carbon dioxide in the second sub-feed stream, and
wherein a molar ratio of methane in the first sub-feed stream is lower than a molar ratio of methane in the second sub-feed stream;
② forming a syngas comprising at least hydrogen and carbon monoxide by supplying the first sub-feed stream, the second sub-feed stream, and steam (H₂O) to a plasma reforming unit,
wherein the first sub-feed stream is configured to be supplied to an upstream end of a plasma induction region defined by a discharge tube of the plasma reforming unit,
wherein the second sub-feed stream and the steam is configured to be supplied to an downstream end of the plasma induction region, and
wherein the first sub-feed stream, the second sub-feed stream and the steam is configured to be reformed by plasma that is generated in the plasma induction region, thereby forming the syngas;
③ forming an intermediate product by supplying the syngas to a gas conversion module,
wherein, in an initial reduction section, the syngas is configured to be supplied to the gas conversion module through a first path in the initial reduction section, and the supplied syngas can be used as a reducing agent for the catalyst within the gas conversion module,
wherein, in a main reaction section, the syngas is configured to be supplied to the gas conversion module through a second path and the supplied syngas can be converted to obtain the intermediate product, and
wherein a pressure of the syngas supplied to the gas conversion module through the first path in the initial reduction section is lower than a pressure of the syngas supplied to the gas conversion module through the second path in the main reaction section; and
④ forming a final product by supplying the intermediate product to a second gas separation module.

2. The method of claim 1,
wherein forming the syngas comprises:
igniting the plasma by supplying seed gas to the upstream end of the plasma induction region and by supplying power to an auxiliary antenna module configured to surround at least a portion of the discharge tube; and
maintaining the plasma discharge by supplying power to a main antenna module configured to surround at least a portion of the discharge tube.

3. The method of claim 2,
wherein the seed gas is configured to be supplied to the plasma induction region from a first time to a second time, and
wherein the first sub-feed stream is configured to be supplied to the plasma induction region from a third time after the first time to a fourth time after the second time.

4. The method of claim 3,
wherein the second time at which the supply of seed gas is stopped is after the time at which power is supplied to the main antenna module.

5. The method of claim 1,
wherein forming the syngas comprises
controlling a flow rate of the first sub-feed stream, a flow rate of the second sub-feed stream and a flow rate of the steam such that a ratio of the methane, carbon dioxide and steam supplied to the plasma induction region is within a predetermined range; and
wherein a molar ratio (H₂/CO) of hydrogen to carbon monoxide in the syngas is configured to be in the range 1.8 to 2.7.

6. The method of claim 1,
wherein a direction in which the first sub-feed stream is supplied to the plasma induction region and a direction in which the second sub-feed stream is supplied to the plasma induction region are configured to be in opposite directions with respect to a central axis of the discharge tube.

7. The method of claim 1, further comprising
performing an additional reaction on a syngas that is formed by plasma reforming by using an additional reaction module configured to be placed in a lower portion of the discharge tube,
wherein the additional reaction module is configured to induce a catalytic reaction with the syngas such that a molar ratio of the hydrogen in the syngas can be increased.

8. The method of claim 1,
wherein the gas conversion module is Water Gas Shift(WGS) module, and
wherein the molar ratio of carbon monoxide in the intermediate product is lower than the molar ratio of carbon monoxide in the syngas.

9. The method of claim 1,
wherein forming an intermediate product comprises:
cooling the syngas transferred to the first path by using a first heat exchanger module in the initial reduction section; and
cooling and pressurizing the syngas transferred to the second path by using a second heat exchanger module and a compressor in the main reaction section.

10. The method of claim 9,
wherein the first heat exchanger is configured to cool the supplied gas in a first temperature range,
wherein the second heat exchanger is configured to cool the supplied gas in a second temperature range, and
wherein a minimum value of the first temperature range is higher than a maximum value of the second temperature range.

11. The method of claim 1,
wherein a first valve is configured to control the flow of the syngas to the first path, and
wherein a second valve is configured to control the flow of the syngas to the second path.

12. The method of claim 11,
wherein forming the syngas comprises,
inducing plasma in the plasma induction region by supplying power in the auxiliary antenna module and the main antenna module placed adjacent to the discharge tube,
wherein an opening time of the first valve is configured to be within a predetermined time from the time at which power is supplied to the main antenna module.

13. The method of claim 11,
wherein a closing time of the first valve is configured to be after a temperature in the gas conversion module satisfies a predetermined temperature.

14. The method of claim 11,
wherein an opening time of the second valve is configured to be after a temperature in the gas conversion module satisfies a predetermined temperature.

15. The method of claim 11,
wherein the closing time of the first valve is configured to be after the opening time of the second valve such that the initial reduction section and the main reaction section are configured to at least partially overlap.

16. The method of claim 1,
wherein forming a final product comprises adsorbing a specific gas from the intermediate product by using an adsorbent in the second gas separation module.
